(19) Europäisches Patentamt
European Patent Office
Office européen des brevets

(11) Veröffentlichungsnummer: **0 399 632 A1**

(12) **EUROPÄISCHE PATENTANMELDUNG**

(21) Anmeldenummer: **90250134.5**

(22) Anmeldetag: **23.05.90**

(51) Int. Cl.5: **C07J 53/00, C07J 71/00, A61K 31/56**

(30) Priorität: **24.05.89 DE 3917274**

(43) Veröffentlichungstag der Anmeldung: **28.11.90 Patentblatt 90/48**

(84) Benannte Vertragsstaaten: **AT BE CH DE DK ES FR GB GR IT LI LU NL SE**

(71) Anmelder: **SCHERING AKTIENGESELLSCHAFT**
**Berlin und Bergkamen**
**Müllerstrasse 170/178 Postfach 65 03 11**
**D-1000 Berlin 65(DE)**

(72) Erfinder: **Ottow, Eckhard, Dr.**
**Moltkestrasse 48**
**D-1000 Berlin 45(DE)**

Erfinder: **Neef, Günter, Dr.**
**Markgraf-Albrecht-Strasse 19**
**D-1000 Berlin 31(DE)**
Erfinder: **Wiechert, Rudolf, Prof. Dr.**
**Petzower Strasse 8A**
**D-1000 Berlin 39(DE)**
Erfinder: **Elger, Walter, Dr.**
**Schorlemer Allee 12B**
**D-1000 Berlin 33(DE)**
Erfinder: **Beier, Sybille, Dr.**
**Uhlandstrasse 121**
**D-1000 Berlin 31(DE)**
Erfinder: **Fritzemeier, Karl-Heinrich, Dr.**
**Rohrwei Strasse 32**
**D-1000 Berlin 27(DE)**

(54) **9alpha-Hydroxy-19,11 beta-überbrückte Steroide, deren Herstellung und diese enthaltende pharmazeutische Präparate.**

(57) Es werden 9α-Hydroxy-19,11β-überbrückte Steroide der allgemeinen Formel I

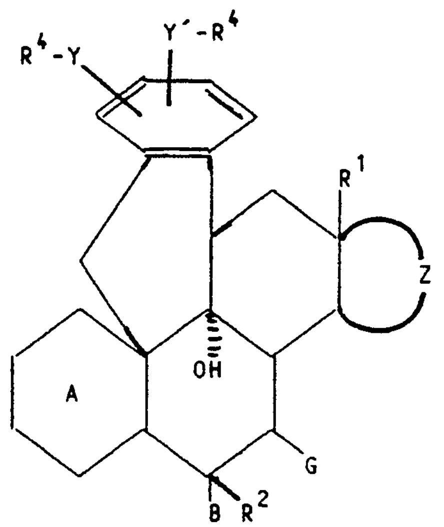

(I),

beschrieben. Die nähere Bedeutung von A, B, $R^1$, $R^2$, G, Z, $R^4$-Y- sowie $R^4$-Y′-ergibt sich aus der Beschreibung. Die Steroide der allgemeinen Formel I besitzen antigestagene und/oder antiglucocorticoide Wirksamkeit und können zur Herstellung von Arzneimitteln verwendet werden.

EP 0 399 632 A1

Xerox Copy Centre

# 9α-Hydroxy-19,11β-überbrückte Steroide, deren Herstellung und diese enthaltande pharmazeutische Präparate

Die vorliegende Erfindung betrifft den in den Patentansprüchen gekennzeichneten Gegenstand, das heißt neue 9α-Hydroxy-19,11β-überbrückte Steroide, Verfahren zu ihrer Herstellung, diese Verbindungen enthaltende pharmazeutische Präparate, ihre Verwendung zur Herstellung von Arzneimitteln sowie die dazu benötigten neuen Zwischenprodukte.

Die erfindungsgemäßen 9α-Hydroxy-19,11β-überbrückten Steroide werden durch die allgemeine Formel I beschrieben

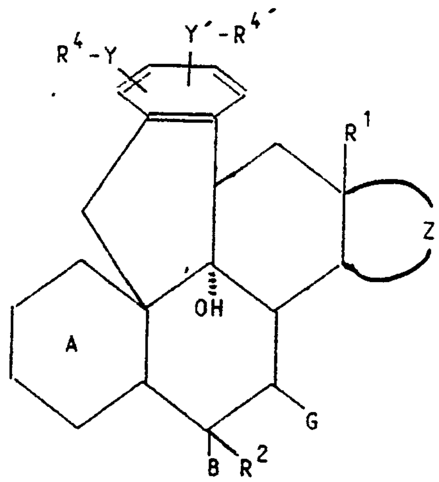

(I),

worin
$R^1$ für einen Methyl- oder Ethylrest,
$R^2$ für ein Wasserstoff-, Chloratom, einen $C_1$-$C_4$-Alkylrest,
B und G, die gleich oder verschieden sind, jeweils für ein Wasserstoffatom, einen $C_1$-$C_4$-Alkylrest oder gemeinsam für eine zweite Bindung zwischen den Kohlenstoffatomen 6 und 7,
B und $R^2$ gemeinsam für eine Methylen- oder Ethylengruppe,
Z für den Rest eines Ringes der Formel

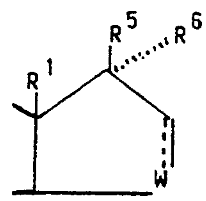

steht, worin
$R^1$ die in Formel I genannte Bedeutung hat,
die von W ausgehende gestrichelte Linie die etwaige Anwesenheit einer Doppelbindung bedeutet,
W einen $CH_2$-, CH-, $CH_2CH_2$- oder $CHCH_2$-Rest,
$R^5$ · $R^6$
$-OR^7$ · $-C{\equiv}C-U$
$-OR^7$ · $-\underset{\underset{O}{\|}}{C}-CH_2-R^8$
$-\underset{\underset{O}{\|}}{C}-CH_2-R^8$ · $-OR^7$
$-\underset{\underset{O}{\|}}{C}-CH_2-R^8$ · $-CH_3$

$-\underset{\underset{O}{\|}}{C}-CH_2-R^8/-H$

$-OR^7/-(CH_2)_m-CH_2-R^9$

$-OR^7/-CH=CH(CH_2)_k-CH_2-R^9$

$-OR^{10}/-H$

$-OR^{10}/-(CH_2)_k-C{\equiv}C-U$

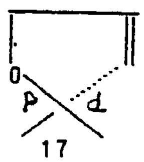
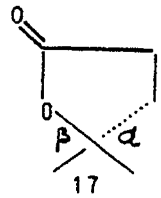
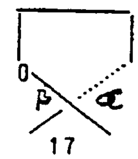

mit $R^7$ in der Bedeutung eines Wasserstoffatoms oder Acylrestes mit 1 bis 4 Kohlenstoffatomen, U in der Bedeutung eines Wasserstoffatoms, einer Alkyl-, Hydroxyalkyl-, Alkoxyalkyl-, Acyloxyalkylgruppe mit jeweils 1 bis 4 Kohlenstoffatomen im Alkyl- bzw. Acylrest oder eines Halogenatoms,

$R^8$ in der Bedeutung eines Wasserstoffatoms, einer Hydroxygruppe, einer Alkyl-, O-Alkyl- oder O-Acylgruppe mit jeweils 1 bis 4 Kohlenstoffatomen,

$R^9$ in der Bedeutung eines Wasserstoffatoms, eines Hydroxy- oder Cyanidrestes, einer O-Alkyl- oder O-Acylgruppe mit jeweils 1 bis 4 Kohlenstoffatomen,

$R^{10}$ in der Bedeutung eines Wasserstoffatoms, einer Alkyl- oder Acylgruppe mit jeweils 1 bis 10 Kohlenstoffatomen,

m in der Bedeutung 0, 1, 2 oder 3,

k in der Bedeutung 0, 1 oder 2,

bedeuten,

worin

$R^4$ und $R^{4'}$, die gleich oder verschieden sind, jeweils für ein Wasserstoffatom, einen Cyanidrest, eine $-OR^{11}$-, $S(O)_kR^{11}$-, $-N(O)_nR^{11}R^{12}$-, $-O-SO_2R^{13}$-, $-P(O)(OR^{14})_2$, $-SiR^{14}_3$ oder $-SnR^{14}_3$ -Gruppe steht mit k in der Bedeutung dei Ziffern 0, 1 oder 2, n in der Bedeutung der Ziffern 0 oder 1,

$R^{11}$ in der Bedeutung eines Wasserstoffatoms oder eines $C_1$-$C_8$-Alkylrestes,

$R^{12}$ in der Bedeutung von $R^{11}$ eines Cyanid- oder eines $C_1$-$C_{10}$-Acylrestes,

$R^{13}$ in der Bedeutung eines perfluorierten $C_1$-$C_4$-Alkylrestes,

$R^{14}$ in der Bedeutung eines $C_1$-$C_4$-Alkylrestes oder

$R^{11}$ und $R^{12}$ innerhalb einer $-N(O)_nR^{11}R^{12}$-Gruppe gemeinsam unter Einschluß von n einen 5- oder 6-gliedrigen heterocyclischen Ring bilden, wobei im Ring noch ein weiteres Heteroatom N, O oder S enthalten sein kann,

Y und $Y'$, die gleich oder verschieden sind, jeweils eine direkte Bindung, eine geradkettige oder verzweigte, gegebenenfalls Doppel- oder Dreifachbindung(en) aufweisende Alkylengruppe mit bis zu 20 Kohlenstoffatomen, die gegebenenfalls substituiert ist durch eine oder mehrere Oxo-, $C_1$-$C_{10}$-Acyloxy-, $-OR^{11}$-, $S(O)_kR^{11}$- und/oder $-N(O)_nR^{11}R^{12}$-Gruppe(n), einen gegebenenfalls substituierten Arylenrest oder

$R^4$-Y und $R^{4'}$-$Y'$ gemeinsam den Rest eines, gegebenenfalls substituierten gesättigten, ungesättigten oder aromatischen 5-oder 6-gliedrigen Ringes mit 0 bis 2 Sauerstoff-, Schwefelatomen und/oder $NR^{11}$-Gruppen bedeuten, mit der Maßgabe, daß nur dann k und n größer als 0 sind, wenn $R^{11}$ für einen $C_1$-$C_8$-Alkylrest steht und

der Ring A für

a)

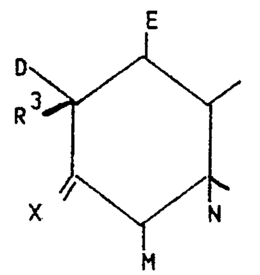

wobei M und N gemeinsam eine zweite Bindung
oder M ein Wasserstoffatom und H eine Hydroxygruppe, wobei dann B, $R^2$, G, $R^3$ D und E Wasserstoffatome sind
und X ein Sauerstoffatom, zwei Wasserstoffatome oder eine Hydroxyiminogruppierung N~OH.
$R^3$ und D, die gleich oder verschieden sind, jeweils ein Wasserstoffatom, einen Nitrilrest oder einen $C_1$-$C_4$-Alkylrest oder gemeinsam eine Methylen-oder Ethylengruppe,
E ein Wasserstoffatom, einen $C_1$-$C_4$-Alkylrest,
D und E gemeinsam eine zweite Bindung, zwischen den Kohlenstoffatomen 1 und 2 oder gemeinsam eine Methylengruppe
bedeuten
oder

b)

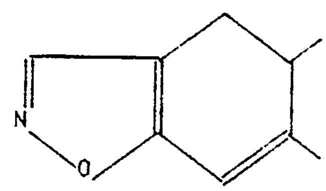

oder

c)

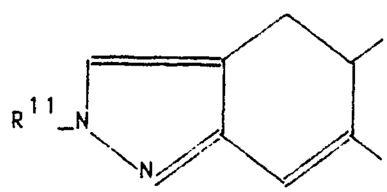

mit $R^{11}$ in der Bedeutung eines Wasserstoffatoms oder eines $C_1$-$C_8$-Alkylrestes stehen. sowie gegebenenfalls deren pharmazeutisch verträgliche Additionssalze mit Säuren.

Wenn Y-$R^4$ = H und Y' die in 1-Position mit einer Oxogruppe substituierte Ethylengruppe und $R^{4'}$ = H ist, liegt als Y'-$^{4'}$ die Acetyl-Gruppe vor, der im Rahmen der Erfindung eine bevorzugte Rolle zukommt.

Bei der Substitution des Phenylringes ist die Monosubstitution in 3-, 4- oder 5-Position sowie die Disubstitution in 4- und 5- oder 3- und 4-Position unter Bildung eines ankondensierten zweiten Ringes, zum Beispiel eines Cyclohexen-, Pyrrol-, Furyl- , Pyrrolin-, 1,3-Dioxacyclopenten-, Pyrazolin-, Didehydromorpholin-, Didehydropiperidin-, Didehydropiperazin-, Dihydropyran- , Pyrimidin-, Pyridin-, Pyrazin-, 1,4-Dioxacyclohexen-Ringes, bevorzugt.

Die für $R^1$ und $R^{11}$ bzw. $R^2$, $R^3$, B, G und D stehenden Alkylreste sollen im Fall von $R^1$ 1 oder 2, im Fall von $R^{11}$ 1 bis 8 und ansonsten 1 bis 4 Kohlenstoffatome tragen, wobei die Methyl-, Ethyl-, Propyl-, Isopropyl-, Butyl- bzw. Methyl-, Ethyl-, Propylgruppen bevorzugt sind.

Steht $R^{12}$ für einen Acylrest, so ist die Formyl-, Acetyl-, Propionyl-, Butyryl und Benzoyl-gruppe bevorzugt.

$R^{11}$ und $R^{12}$ stehen auch gemeinsam unter Einschluß des Stickstoffatoms für einen heterocyclischen Fünf- oder Sechsring, der außer N- und C-Atome auch noch zusätzlich ein O- oder S-Atom enthalten kann; beispielsweise genannt seien der Pyrrol-, Pyrrolidin-, Piperidin-, Piperazin-, Morpholin-, Oxa- und

Thiazolidin-sowie Thiadiazolidin-Ring.

Die in $R^5$ und $R^6$ bzw. $R^7$, $R^8$, $R^9$, $R^{10}$ und U der allgemeinen Formel I enthaltenen Alkyl-, Alkoxy- sowie Acyloxygruppen sollen jeweils 1 bis 4 Kohlenstoffatome enthalten, wobei die Methyl-, Ethyl-, Propyl-, Isopropyl-, Methoxy-, Ethoxy-Propoxy-, Isopropoxy-, Formyl-, Acetyl-, Propionyl- und Isopropionylgruppe bevorzugt sind.

Von den Alkenylresten in $R^6$ sind die Propenyl- und Butenylgruppe, die in der E-oder Z-Konfiguration vorliegen können, bevorzugt, d.h. wenn $R^6$ für $-CH=CH-(CH_2)_kCH_2-R^9$ steht, dann soll k bevorzugt 0 oder 1 bedeuten.

Die neuen Verbindungen der allgemeinen Formel I werden erfindungsgemäß nach dem Verfahren gemäß Anspruch 13 hergestellt.

Zur Herstellung der Zwischenprodukte der allgemeinen Formel II

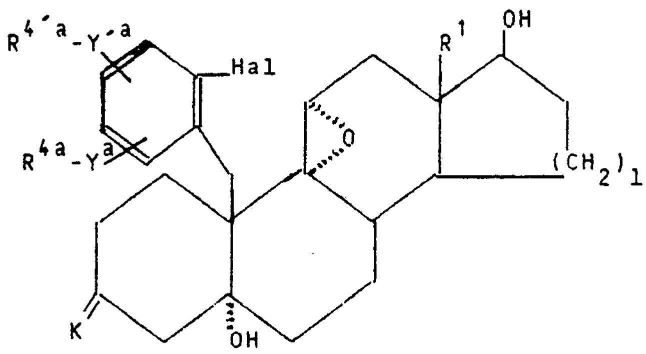

(II),

worin

$R^1$ einen Methyl- oder Ethylrest,

l die Ziffern 1 oder 2,

K eine in Form des Ketals oder Thioketals blockierte Ketogruppe bedeuten, und

Hal ein Chlor-, Brom- oder Jodatom bedeutet,

$R^{4a}$, $R^{4'a}$, $Y^a$ und $Y'^a$ unter Ausschluß des Cyanidrestes die gleiche Bedeutung wie $R^4$, $R^{4'}$, Y und Y′ haben, wobei gegebenenfalls vorhandene Hydroxy-, Mercapto-, Amino-, Oxo- und/oder terminale Acetylengruppen geschützt sind, die ebenfalls Gegenstand der Erfindung sind, geht man aus von den in der Europäischen Patentanmeldung EP-A 0283 428 beschriebenen Verbindungen der Formel IIIa

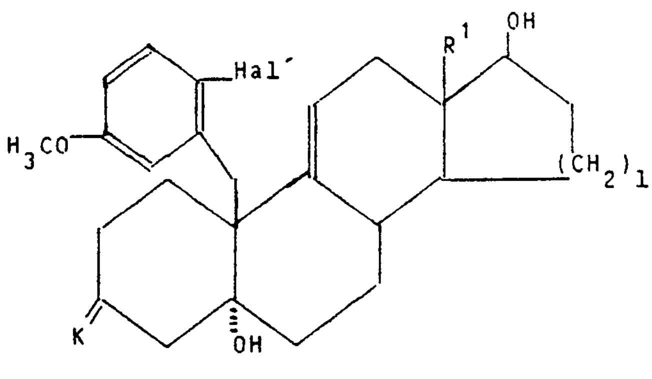

(IIIa),

worin

$R^1$ und K die oben genannte Bedeutung haben und Hal′ für ein Chlor- oder Bromatom steht oder von den nach ebenfalls in der EP-A 0283428 enthaltenen Vorschriften erhältlichen Verbindungen der allgemeinen Formel IIIb

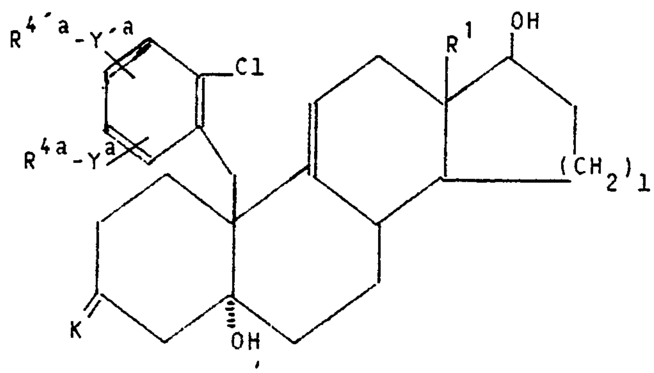

(IIIb),

worin $R^{4'a}$, $Y'^a$, $R^{4a}$, $Y^a$ sowie K, 1 und $R^1$ die bereits vorstehend genannten Bedeutungen haben.
In den Verbindungen der allgemeinen Formeln IIIa bzw. IIIb ist im allgemeinen vor der weiteren Umsetzung das in 2-Position des Phenylringes stehende Chloratom beispielsweise unter den im Beispiel 8a), b) genannten Bedingungen gegen ein Brom- (oder Jod-) atom auszutauschen.

Im nächsten Schritt wird dann eine Verbindung der allgemeinen Formel IIIa oder IIIb durch Epoxidierung der 9,11-Doppelbindung, beispielsweise durch Umsetzung mit einer Persäure, in eine Verbindung der allgemeinen Formel II überführt, etwa nach den in J. Org. Chem., 38, No. 12, S. 2267 (1973) oder J. Org. Chem., 44, No. 8, S. 1351 (1979) beschriebenen Vorschriften. Als Epoxidierungsmittel dient vorzugsweise m-Chlorperbenzoesäure.

Nach Schutz der gegebenenfalls im 19-Phenylring vorhandenen funktionellen Gruppen werden die neuen Verbindungen der allgemeinen Formel II einer Cyclisierung unterworfen.

Die von K umfaßten Hydroxy-, Mercapto- und Keto-Schutzgruppen sind im sauren Milieu leicht abspaltbare Gruppen, wie zum Beispiel die Methoxymethyl-, Ethoxymethyl- , Tetrahydropyranyl-, Ethylendioxyketal-, Ethylendithioketal- oder 2,2-Dimethyltrimethylendioxyketalgruppe. Eine (oder mehrere) am 19-Phenylring vorhandene Hydroxygruppe(n) wird (werden) durch eine basisch zu entfernende Gruppe, beispielsweise durch eine Methoxygruppe, geschützt. Diese kann beispielsweise durch Reaktion mit Natriumthiophenolat wieder abgespalten werden. Schutzgruppen für Amino- und terminale Acetylengruppen (zum Beispiel die Trimethylsilyl- und tert.-Butyldimethylsilylgruppe) sind dem Fachmann ebenfalls bekannt und werden nach der gewünschten Reaktionsfolge auch nach literaturbekannten Verfahren gespalten [Synthesis 1980, 627, J. Org. Chem. 46 (1986) 2280]

Die Umwandlung von 11 zu den neuen 9α-Hydroxy-19, 11β-überbrückten Steroiden der allgemeinen Formel IVb

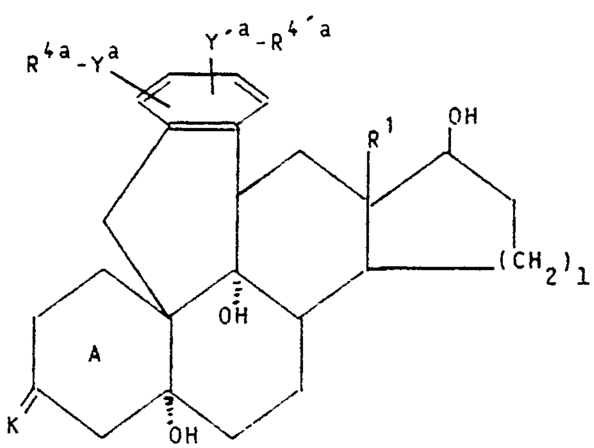

(IVb),

worin $R^1$, K und l sowie $R^{4'a}$, $Y'^a$, $R^{4a}$, $Y^a$ die oben genannte Bedeutung haben und die ebenfalls Gegenstand der Erfindung sind, erfolgt über ein Arylanion als reaktive Spezies, welches sich nach einem Halogen-Metall-Austausch des ortho-Chlor-, Brom- (oder Jod-) atoms im 19-Phenylring der Verbindung der

allgemeinen Formel II gegen ein $Me^{\oplus}$ -, $MgX^{\oplus}$ -, $Cu^{\oplus}$ - oder $CuX^{\oplus}$ -Ion ($Me^{\oplus}$ = Alkaliion, X = Cl, Br, J) bilden kann.

Beispielsweise wird n-Butyllithium (vergl. Beispiel 1b), t-Butyllithium oder metallisches Lithium in einem inerten Lösungsmittel wie etwa Diethylether verwendet, um den Halogen-Metall-Austausch und die sich daran anschließende Cyclisierung zu einer Verbindung der allgemeinen Formel IVb zu bewirken.

Daß hierbei ein 6-gliedriger Ring (Ringschluß zum 11-C-Atom) und nicht ein 5-gliedriger Ring (Ringschluß zum 9-C-Atom) sich bildet, mußte überraschen. In einem ähnlichen Fall war ausschließlich die Bildung eines 5-gliedrigen Ringes beobachtet worden (vergl. J. Org. Chem., 43, No. 19, S. 3800 (1978) und die Baldwin-Regeln in J. Chem. Soc., Chem. Commun. 734 (1976)).

Die Umwandlung der so erhaltenen Cyclisierungsprodukte in die letzlich gewünschten Endprodukte der allgemeinen Formel I erfolgt analog literaturbekannten Verfahren (zum Beispiel J. Fried, J.A. Edwards, "Organic Reactions in Steroid Chemistry", Van Nostrand Reinhold Company 1972, Vol. 1 und 2; "Terpenoids and Steroids", Specialist Periodical Report, The Chemical Society, London, Vol. 1-12) indem man entweder zuerst

a) die C-17-Hydroxygruppe oxidiert und anschließend

b) gegebenenfalls eine eine Schutzgruppe aufweisende Hydroxygruppe im 19,11β-Phenylenring von dieser Schutzgruppe befreit, gewünschtenfalls aus der Hydroxyverbindung ein entsprechendes Perfluoralkylsulfonat ($C_1$-$C_4$) herstellt, gegebenenfalls das Perfluoralkylsulfonat entweder direkt oder durch Austausch der Perfluoralkylsulfonatabgangsgruppe gegen eine Zinntrialkylgruppe über die entsprechende Zinntrialkylverbindung in eine Verbindung überführt, die im 19,11β-Phenylenring, gegebenenfalls nach weiteren Reaktionen, das gewünschte Substitutionsmuster aufweist, oder zuerst b) und dann a) ausführt und danach

c) den Ring D in gewünschter Weise nach an sich bekannten Methoden funktionalisiert, das so erhaltene Produkt der Einwirkung eines Dehydratationsmittels, das auch zur Freisetzung der 3-Oxogruppe befähigt ist, zur Wasserabspaltung unter gleichzeitiger Ausbildung der 4(5)-Doppelbindung unterwirft, wobei gewünschtenfalls die Freisetzung der 3-Oxogruppe und die Wasserabspaltung nacheinander stattfinden, und anschließend, gegebenenfalls nach erneutem Schutz von intermediär freigesetzten, im 19,11β-Phenylenring und/oder Z enthaltenen funktionellen Gruppen, die gewünschten Funktionen der Ringe A und B des Steroidgerüstes einführt oder

d) das so erhaltene Produkt der Einwirkung eines Dehydratationsmittels, das auch zur Freisetzung der 3-Oxogruppe befähigt ist zur Wasserabspaltung unter gleichzeitiger Ausbildung der 4(5)-Doppelbindung unterwirft, die gewüns chten Funktionen der Ringe A und B des Steroidgerüstes einführt und anschließend, nach Schutz der 3-Oxogruppe, den Ring D in gewünschter Weise funktionalisiert, oder die Schritte a) und b) nach dem Schritt c) oder d) ausführt,

das so erhaltene Produkt gegebenenfalls von Schutzgruppen befreit, gewünschtenfalls die im 19,11β-Phenylenring gegebenenfalls enthaltene(n) Hydroxy- , Mercapto- und/oder Aminogruppe(n) alkyliert bzw. acyliert, gewünschtenfalls einen Cyanidrest in den/die Arylsubstituenten einführt, gewünschtenfalls die in dem(n) Arylsubstituenten gegebenenfalls enthaltene(n) Amino- und/oder Sulfidgruppe(n) oxidiert, gewünschtenfalls mit Hydroxylamin-hydrochlorid zum Produkt der allgemeinen Formel I mit X in der Bedeutung der Hydroxyiminogruppierung N~OH umsetzt sowie gegebenenfalls ein pharmazeutisch verträgliches Säureadditionssalz herstellt.

Im Verlaufe dieser Reaktionswege kann es notwendig werden, intermediär erneut Schutzgruppen in Zwischenprodukte, zum Beispiel für in Z enthaltene funktionelle Gruppen bei anschließender Funktionalisierung der Ringe A und B oder für die 3-Ketogruppe bei anschließendem Aufbau des Ringes D, einzuführen.

Die für die Herstellung fast aller Endprodukte nötige Oxidation der 17β-Hydroxygruppe wird in an sich bekannter Weise, zum Beispiel durch Oppenauer-Oxidation oder mit Chromsäurereagenzien (Jones'-Reagenz oder Chromsäure-Pyridin) durchgeführt.

Die Freisetzung der 3-Ketofunktion unter gleichzeitiger Wasserabspaltung und Ausbildung der 4(5)-Doppelbindung erfolgt durch Behandlung mit Säure oder einem sauren Ionenaustauscher. Die saure Behandlung erfolgt in an sich bekannter Weise, indem man das entsprechende 5α-Hydroxy-3-ketal in einem mit Wasser mischbaren Lösungsmittel, wie wäßrigem Methanol, Ethanol oder Aceton, löst und auf die Lösung katalytische Mengen Mineral- oder Sulfonsäure, wie Salzsäure, Schwefelsäure, Phosphorsäure, Perchlorsäure oder p-Toluolsulfonsäure, oder eine organische Säure, wie Essigsäure, so lange einwirken läßt, bis vorhandene Schutzgruppen entfernt sind und gegebenenfalls Wasser abgespalten ist. Die Umsetzung, die bei Temperaturen von 0 bis 100 °C abläuft, kann auch mit einem sauren Ionenaustauscher vorgenommen werden. Der Verlauf der Umsetzung kann mit analytischen Methoden, beispielsweise durch Dünnschichtchromatographie entnommener Proben, verfolgt werden.

Im allgemeinen wird die Schutzgruppenentfernung und Wasserabspaltung in einem Reaktionsschritt bewerkstelligt, indem man das entsprechende 5α-Hydroxy-3-ketal bzw. 5-en-3-Ketal in stark saurem

Medium eine gewisse Zeit lang reagieren läßt, wie dies in Beispiel 1i) beschrieben ist. Genausogut ist es aber erfindungsgemäß möglich, die Schutzgruppenentfernung und Wasserabspaltung in zwei voneinander getrennten Reaktionsschritten durchzuführen, indem zuerst durch eine kürzere Behandlung des entsprechenden 5α-Hydroxy-3-ketals in mäßig saurem Medium und gegebenenfalls bei einer Temperatur von 0°C oder darunter zunächst die entsprechende 5α-Hydroxy-3-keto-Verbindung gewonnen und gegebenenfalls isoliert wird, wie dies exemplarisch in Beispiel 8 gezeigt ist. Die 5α-Hydroxy-3-keto-Verbindung wird dann durch weiteres Einwirkenlassen von Säure unter Wasserabspaltung in die 3-Keto-4-en-Verbindung überführt.

Die selektive Entfernung der Schutzgruppe ohne Wasserabspaltung gelingt in manchen Fällen auch im stark sauren Medium (z.B. 4n HCl), wenn nur kurzzeitig bei 0°C oder darunter reagieren gelassen wird.

Ein ganz besonderer Vorteil der vorliegenden Erfindung liegt in der großen Bandbreite der am 19-Phenylrest einführbaren Substituenten (M. Pereyre, J.-P. Quintard, A. Rahm, Tin in Organic Synthesis: Butterworths, 1987). Zum einen können die im späteren Endprodukt vorhandenen Substituenten $R^4$-Y- bzw. $R^{4'}$-Y'-über eine Verbindung der allgemeinen Formel IIIb direkt eingeführt werden, (vergl. EP-A 0283 428).

Mit einer weiteren Ausführungsform des erfindungsgemäßen Verfahrens gelingt es, den (die) Substituenten im 19,11β-Phenylenrest über einen weiten Bereich zu variieren, indem der (die) Substituent(en) erst nach der Cyclisierung, und zwar vor, gleichzeitig mit oder erst nach Fertigstellung der Struktur der Ringe A, B und D eingeführt wird (werden). Dazu wird mindestens eine der im 19,11β-Phenylenrest in der Formel IVb vorhandenen und geschützten Hydroxygruppe von ihrer Schutzgruppe befreit und aus der freien OH-Verbindung durch Umsetzung mit Perfluoralkylsulfonsäureanhydrid (Alkyl = $C_1$-$C_4$) nach an sich bekannten Methoden [P.J. Stang, M. Hanack and L.R. Subramanian, Synthesis 85 (1982)] die entsprechende Perfluoralkylsulfonatverbindung, hergestellt.

Dabei wird entweder so vorgegangen, daß in einer übergangsmetallkatalysierten Reaktion (vorzugsweise Pd°) die Perfluorsulfonatabgangsgruppe unter im wesentlichen fast gleichzeitiger Substitution durch den gewünschten Substituenten oder dessen Vorstufe verdrängt wird (J.E. McMurry and S. Mohanraj, Tetrahedron Letters, 24, No. 27, S. 2723-2726, 1983; X. Lu und J. Zhu, Communications, S. 726-727, 1987; Q. -Y. Chen und Z. -Y. Yang, Tetrahedron Letters 27, No. 10, S. 1171-1174, 1986; S. Cacchi, P.G. Ciattini, E. Morera und G. Ortar, Tetrahedron Letters, 27, No. 33, S. 3931-3934, 1986; A.M. Echavarren und J.K. Stille, J. Am. Chem. Soc. 1987, 109, S. 5478-5486) oder es wird aus der Perfluoralkylsulfonat-Verbindung intermediär und übergangsmetallkatalysiert eine entsprechende Tri-organylstannyl- , vorzugsweise Tri-n-alkylstannyl-Verbindung hergestellt [J.K. Stille, Angew. Chem. 98 (1986), S. 504-519]. Diese wird anschließend mit einem halogen-, vorzugsweise brom- oder jodsubstituierten carbocyclischen (vergl. Beispiel 6c) oder heterocyclischen Aromaten (vergl. Beispiel. 3b) [Y. Yamamoto, Y. Azuma, H. Mitoh, Communications, S. 564-565, 1986; T. J. Bailey, Tetrahedron Letters, 27, No. 37, S. 44074410, 1986], der gegebenenfalls noch weitere Substituenten tragen kann, umgesetzt; der 19,11ß-Phenylenrest weist darin dann die gewünschte bzw. einen Vorläufer der gewünschten Substitution auf. Die Vorläufer werden nach gängigen Methoden der organischen Chemie zu den letztendlich gewünschten Verbindungen weiterverarbeitet.

Weist der 19-Phenylring zwei geschützte Hydroxygruppen auf, so kann zunächst lediglich die Schutzgruppe der einen (ersten) Hydroxygruppe selektiv entfernt werden, die freie Hydroxygruppe funktionalisiert werden, anschließend gegebenenfalls die Schutzgruppe der zweiten Hydroxygruppe abgespalten und diese Hydroxygruppe modifiziert werden, gegebenenfalls auch durch Reaktion mit der sich an der ersten Hydroxygruppe nunmehr befindenden Funktion.

Die Einführung von 1,2 und/oder 6,7-Doppelbindungen neben der 3,4-Doppelbindung gelingt nach bekannten Methoden, zum Beispiel mit Dehydrierungsmitteln wie Selendioxid, Chloranil, Thalliumtriacetat oder Dichlordicyanobenzochinon (DDQ) bzw. durch Allyl- oder Dienoletherbromierung und anschließende Bromwasserstoffabspaltung. [J. Fried, J.A. Edwards, Organic Reactions in Steroid Chemistry, Van Nostrand Reinhold Company 1972, S. 265-374, 1; Tetrahedron 42, (1986) 2971]

Die Allylbromierung wird zum Beispiel mit N-Bromsuccinimid, N-Bromacetamid, 1,3-Dibrom-5,5-dimethylhydantoin oder Dibromtetrachlorethan in Gegenwart eines Radikalbildners wie Dibenzoylperoxid in einem Lösungsmittel vorgenommen. Als Lösungsmittel kommen aprotische Lösungsmittel wie Dioxan und chlorierte Kohlenwasserstoffe, wie zum Beispiel Tetrachlorkohlenstoff, Chloroform oder Tetrachlorethylen infrage. Die Umsetzung erfolgt zwischen 0°C und der Siedetemperatur der Lösung.

Die Dienoletherbromierung wird zum Beispiel analog der Vorschrift in Steroids I, 233 durchgeführt.

Die Bromwasserstoffabspaltung unter Ausbildung der $\Delta^6$- Doppelbindung erfolgt durch Erhitzen der 6-Bromverbindung mit basischen Mitteln, vorzugsweise mit Lithiumbromid und Lithiumcarbonat oder mit Lithiumbromid und Calciumcarbonat in einem aprotischen Lösungsmittel wie Dimethylformamid bei Temperaturen von 50 bis 120°C. Eine weitere Möglichkeit der HBr-Abspaltung besteht darin, daß man die 6-Bromverbindung in Collidin oder Lutidin erhitzt.

Ausgehend von einem gesättigten Ring A können Doppelbindungen in 1,2- und 4,5-Stellung gleichzeitig eingeführt werden, zum Beispiel durch Bromierung zum 2,4-Dibrom-3-keton und Dehydrobromierung des Dibromids mit zum Beispiel Lithium-oder Calciumcarbonat und Lithiumbromid in Dimethylformamid.

Die Einführung einer 6-Methylengruppe kann zum Beispiel ausgehend von einem 3-Amino-3(4),5(6)-dien-Derivat durch Umsetzung mit Formalin in alkoholischer Lösung (Helv. Chim. Acta. 56 (1973) 2396) zur 6α-Hydroxymethylgruppe und anschließender saurer Wasserabspaltung zum Beispiel mit Salzsäure in Dioxan/Wasser oder ausgehend von einem 3-Alkoxy-3(4),5(6)-dien-Derivat, analog der im US-Patent 4,544,555 beschriebenen Methode oder direkt, ausgehend von einem 3-Oxo-4(5)-en-Derivat analog der Vorschrift in Synthesis (1982) 34 erfolgen.

Die Methylenierung der 6-Methylen- zur 6,6-Ethylenverbindung erfolgt mit Dimethylsulfoxoniummethylid. Hierzu wird das 6-Methylen-Steroid zu einer Suspension von Trimethylsulfoxoniumjodid mit Natriumhydrid in Mineralöl und Dimethylsulfoxid oder zu einer Lösung von Trimethylsulfoxoniumjodid und Natriumhydroxid in Dimethylsulfoxid gegeben. Die Reaktion ist nach 15 bis 60 Minuten bei 20 bis 40 °C beendet (J.Am.Chem.Soc. 84(1962) 866; Europäische Patentanmeldung 0150157) Die Einführung einer 2-Methylengruppe erfolgt analog der Methode von A. J. Manson und D. Wood [J. Org. Chem. 32 (1967) 3434] oder den dort zitierten Methoden.

Die Methylenierung der 2-Methylen- zur 2,2-Ethylenverbindung erfolgt analog der Methylenierung der 6-Methylenverbindung [siehe auch Chem. Ber. 98 (1965) 1470].

Mono- bzw. dialkylierte Verbindungen in 2-Position können zum Beispiel analog der Methode von L. Nedelec, Tetrahedron 30 (1974) 3263 erhalten werden.

Alkylierte Verbindungen in Position 1 bzw. 7 werden durch 1,4- bzw. 1,6-Addition an die entsprechenden Enone nach bekannten Methoden (J. Fried, J.A. Edwards: Organic Reactions in Steroid Chemistry, Van Nostrand Reinhold Company 1972, Seite 75 bis 82, 2; und J. Am. Chem. Soc. 99 (1977) 1673) erhalten.

Alkylierte Verbindungen in Position 6 können zum Beispiel durch Öffnung der entsprechenden 5α,6α-Epoxide und Folgereaktionen erhalten werden (J. Fried, J.A. Edwards: Organic Reactions in Steroid Chemistry, Van Nostrand Reinhold Company 1972, Seite 82-86, 2).

1α,2α-, 6α,7α-, 6β,7β-Methylenverbindungen oder eine Kombination des 1α,2α,Methylenstrukturelements mit den beiden 6,7-Methylen-Strukturelementen lassen sich durch Addition von Diazomethan oder Dimethylsulfoxoniummethylid an die entsprechenden Enone bzw. durch Simmons-Smith-Reaction (J. Fried, J.A. Edwards: Reactions in Steroid Chemistry, Van Nostrand Reinold Company 1972, Seite 100-126; Rev. Soc. Quim. Mex. (1969) 171A; Chem. Ber. 101 (1968) 935, Chem. Ber. 99 (1966) 1118; Zeitschr. f. Naturf. 19b (1964) 944) der entsprechenden Allylalkohole erhalten.

Die Herstellung des an die Positionen 2 und 3 annelierten Isoxazolringes erfolgt über die Synthese der 2-Hydroxymethylenverbindungen [Steroids 6 (1962) 178; J. Amer. Chem. Soc. 83 (1961) 1478] und ihre Umsetzung mit Hydroxylamin [J. Med. Chem. 6 (1963) 1].

[2,3-d]isoxazole sind auch gute Ausgangsmaterialien für die Synthese von 2-Cyano-Steroiden [J. Med. Chem. 6 (1963) 1].

Die Herstellung des an die Positionen 2 und 3 annelierten Pyrazolringes erfolgt durch Umsetzung von 2-Hydroxymethylen-3-oxo-Edukten mit $R^{11}$-substituiertem Hydrazin (US-Patent 3,704,295).

Die Einführung des Chlor- bzw. Methyl-Substituenten in C-6 des Steroidgerüsts gelingt z.B. durch die in der Deutschen Auslegeschrift 1,158,966 bzw. in US-Patent 4,544,555 und US-Patent 4,196,203 angegebenen Methoden über die entsprechenden 6,7-Epoxide bzw. 6-Methylen-Derivate sowie durch Oxidation des 6-Chlor-3,5-dienolethers mit Dichlordicyanobenzochinon (DDQ) unter sauren Bedingungen [Belgisches Patent 621,197 (1962)].

Die Entfernung der 3-Oxogruppe zu einem Endprodukt der allgemeinen Formel I mit X in der Bedeutung von zwei Wasserstoffatomen kann z.B nach der in DOS 2805490 angegebenen Vorschrift durch Thioketalisierung und anschließende reduktive Spaltung erfolgen.

Edukte mit einem D-Homo-Steroidgerüst sind auch z.B. durch Tiffeneau-Umlagerung analog der in Australian 3. Chem. 8 (1955), 519 und in "Organic Reactions in Steroid Chemistry" Vol 2, 388 veröffentlichten Vorschrift zu erhalten. Die notwendigen 17α-Aminomethyl-17β-hydroxyverbindungen sind zum Beispiel über die Öffnung der 17,20-Spiroepoxide mit Ammoniak oder auch durch Lithiumaluminiumreduktion der acetylierten 17β-Hydroxy-17α-cyanoverbindung zugänglich. Die Spiroepoxide sind durch Umsatz der entsprechenden 17-Ketone mit Diemthylsulfoniummethylid in Dimethylformamid [Journal f. prakt. Chemie 314-(1972), 667-668) zugänglich. Die acetylierten Cyanhydrine sind durch Anlagerung von Cyanwasserstoff an die entsprechenden 17-Ketone und anschließende Acetylierung nach bekannten Vorschriften (z.B. Australian J. Chem. 8 (1955),519) zugänglich.

Edukte mit einem ungesättigten D-Ring sind zum Beispiel durch modifizierte Saegusa-Oxidation (Tetrahedron 42 (1986) 2971) der entsprechenden Enolverbindungen des 17-Ketons zugänglich. Zum

Beispiel ist der Trimethylsilylenolether durch Überführung des 17-Ketons mit Lithiumdiisopropylamid in Tetrahydrofuran in das korrespondierende Enolat und Abfang durch Trimethylchlorsilan darstellbar (Synthesis 1983, 1).

Die Einführung der Substituenten $R^5$ und $R^6$ erfolgt nach den üblichen Verfahren des C-17-Seitenkettenaufbaus durch nucleophile Addition an das - durch z.B. Oppenauer-Oxidation der C-17-Hydroxygruppe erhaltene -17-Keton und Folgereaktionen ("Terpenoids and Steroids", Specialist Periodical Report, The Chemical Society, London, Vol. 1-12)

Die Einführung des Substituenten -C≡C-U als $R^6$, wobei U die oben angegebene Bedeutung hat, erfolgt mit Hilfe einer Verbindung der allgemeinen Formel MC≡C-U`, in der U eine Alkin-Schutzgruppe, wie zum Beispiel der Trimethylsilyl- oder tert.-Butyldimethylsilylrest, oder aber im Fall, wenn U eine Alkylgruppe mit 1-4 C-Atomen ist, U` selbst der Rest U ist.

Die metallorganische Verbindung kann auch in situ gebildet und mit dem 17-Keton zur Reaktion gebracht werden. So kann man zum Beispiel auf das 17-Keton in einem geeigneten Lösungsmittel Acetylen und ein Alkalimetall, insbesondere Kalium, Natrium oder Lithium, in Gegenwart eines Alkohols oder in Gegenwart von Ammoniak einwirken lassen. Das Alkalimetall kann auch in Form von zum Beispiel Methyl- oder Butyllithium zur Einwirkung kommen. Als Lösungsmittel sind insbesondere Dialkylether, Tetrahydrofuran, Dioxan, Benzol und Toluol geeignet.

Die Einführung von 3-Hydroxypropin, -propen bzw. -propan in 17-Stellung erfolgt durch Umsetzung des 17-Ketons mit dem Dianion des Propargylalkohols (3-Hydroxypropin), zum Beispiel dem in situ generierten Dikaliumsalz des Propargylalkohols, zum 17α-(3-Hydroxyprop-1-inyl)-17β-hydroxyderivat oder mit metallierten Derivaten des 3-Hydroxypropins, zum Beispiel mit 1-Lithium-3-(tetrahydropyran-2′-yloxy)-prop-1-in-1-id, zum 17-[3-(Tetrahydropyran-2′-yloxy)-prop-1-inyl]-17β-hydroxyderivat, die anschließend zu den 17-(3-Hydroxypropyl- bzw. Hydroxypropenyl)17β-hydroxy-Verbindungen hydriert werden können. Das gelingt zum Beispiel durch Hydrierung bei Raumtemperatur und Normaldruck in Lösungsmitteln wie Metanol, Ethanol, Propanol, Tetrahydrofuran (THF) oder Essigester unter Zusatz von Edelmetall-Katalysatoren wie Platin oder Palladium.

Die Einführung homologer Hydroxyalkin-, Hydroxyalken- und Hydroxyalkangruppen erfolgt in entsprechender Weise mit Homologen des Propargylalkohols.

Die Verbindung mit der Z-konfigurierten Doppelbindung in der Hydroxypropenylgruppe entsteht durch Hydrieren der acetylenischen Dreifachbindung mit einem desaktivierten Edelmetallkatalysator (J. Fried, J.A. Edwards: Organic Reactions in Steroid Chemistry, Van Nostrand Reinhold Company 1972, Seite 134; und H.O. House: Modern Synthetic Reactions 1972, Seite 19).Als desaktivierte Edelmetallkatalysatoren kommen beispielsweise 10% Palladium auf Bariumsulfat in Gegenwart eines Amins oder 5 % Palladium auf Calciumcarbonat unter Zusatz von Blei(II)-acetat infrage. Die Hydrierung wird nach der Aufnahme von einem Äquivalent Wasserstoff abgebrochen.

Die Verbindung mit der E-konfigurierten Doppelbindung in der Hydroxypropenylgruppe entsteht durch Reduktion der acetylenischen Dreifachbindung in an sich bekannter Weise. In der Literatur sind eine ganze Reihe von Methoden zur Umwandlung von Alkinen in trans-Olefine beschreiben, beispielsweise die Reduktion mit Natrium in flüssigem Ammoniak (J. Am. Chem. Soc. 63 (1941) 216), mit Natriumamid in flüssigem Ammoniak (J. Chem. Soc. 1955) 3558), mit Lithium in niedermolekularen Aminen (J. A. Chem. Soc. 77 (1955) 3378), mit Boranen (J. Am. Chem. Soc. 93 (1971) 3395 und 94 (1972) 6560), mit Diisobutylaluminiumhydrid und Methyl-Lithium (J. Am. Chem. Soc. 89 (1967) 5085) und insbesondere mit Lithiumaluminiumhydrid/Alkoholat (J. Am. Chem. Soc. 89 (1967) 4245). Eine weitere Möglichkeit ist die Reduktion der Dreifachbindung mit Chrom(II)-sulfat in Gegenwart von Wasser oder Dimethylformamid in schwach saurem Milieu (J. Am. Chem. Soc. 86 (1964) 4358) sowie allgemein die Reduktion durch Einwirkung von Übergangsmetallverbindungen unter Wechsel der Oxydationsstufe.

Die Einführung der Hydroxyalkene kann auch direkt erfolgen durch Addition einer entsprechenden metallierten Hydroxyalkenylverbindung, wie zum Beispiel 1-Lithium-3-(tetrahydropyran-2′-yloxy)-prop-1(E)-en (J. Org. Chem. 40 2265) oder 1-Lithium-3-(tetrahydropyran-2′-yloxy)-prop-1(Z)-en (Synthesis 1981, 999) Homologe können auf diese Art ebenfalls eingeführt werden.

Die Einführung von 3-Hydroxypropan in 17-Stellung kann ebenfalls direkt durch Umsetzung des 17-Ketons mit metallierten Derivaten von 3-Halogen-propanolen - wobei die Hydroxygruppe im Metallierungsschritt als Alkoholat (Tetrahedron Letters 1978, 3013) oder als geschützte Funktion (J. Org. Chem. 37, 1947) vorliegt - zu der 17-(3-Hydroxypropyl)-17β-hydroxy-verbindung bzw. zu der an der terminalen Hydroxygruppe geschützten Verbindung erfolgen. Als Schutzgruppen kommmen zum Beispiel die Ethoxyethyl-, Tetrahydropyranyl- und Methoxymethyl-Gruppen in Frage.

Werden Endprodukte der Formel I gewünscht mit $R^5$ $R^6$ in dar Bedeutung von

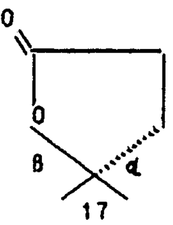

50 wird die 17-(3-Hydroxypropyl)-Verbindung in an sich bekannter Weise oxydiert, zum Beispiel mit Jones' Reagenz, Braunstein, Pyridiniumdichromat, Pyridiniumchlorochromat, Chromsäure-Pyridin oder dem Fetizon-Reagenz Silbercarbonat/Celite (Compt. rend. 267 [1968] 900).

Die von Endprodukten der Formel I mit $R^5/R^6$ in der Bedeutung von

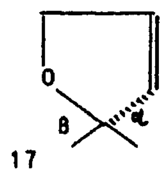

erfolgt durch Ringschlußreaktion des entsprechenden 17-(3-Hydroxyprop-1-(Z)-enyl-17-β-hydroxy-Eduktes.

Sollen $R^5/R^6$ gemeinsam für

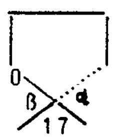

stehen, so wird entweder eine vorstehend beschriebene Dihydrofuranverbindung katalytisch hydriert oder die entsprechende 17-(3-Hydroxypropyl)-17β-hydroxyverbindung cyclisiert.

Der Aufbau der 17-Cyanmethylseitenkette erfolgt in an sich bekannter Weise aus dem 17-Keton zum Beispiel über das 17-Spiroepoxid und Spaltung des Spiroepoxids mit HCN gemäß Z. Chem. 18 (1978) 259-260.

Auch die Einführung der 17-Hydroxyacetylseitenkette erfolgt nach an sich bekannten Methoden, beispielsweise nach den in J. Org. Chem. 47 (1982), 2993-2995, Chem.Ber. 113 (1984), 1184 bzw. US-Patent 4.600.538 beschriebenen Methoden.

Zur Einführung der Gruppierungen

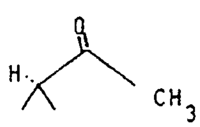

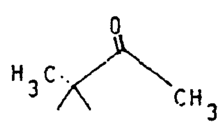

wird das 17-Keton mit Tosylmethylisocyanid (Chem. Ind. 1972 213) in die 17-Nitrilverbindung (Tetrahedron 31(1975),2151) überführt, das direkt mit Methyllithium oder Methylmagnesiumbromid in die 17-Acetylverbindung umgewandelt werden kann, welche nach Enolisierung mit K-tert.-Butylat in Tetrahydrofuran und Umsetzung mit Methyljodid die gewünschte 17α-Methyl-17βacetylgruppierung liefert. Diese Sequenz Methyladdition an das Nitril und anschließende Alkylierung kann auch in umgekehrter Folge ausgeführt werden.

In Z bzw. im 19,11β-Phenylenring vorhandene freie Hydroxy- bzw. Hydroxy-, Mercapto- und/oder Aminogruppen können in an sich bekannter Weise alkyliert oder acyliert werden.

Im 19,11β-Phenylenring enthaltene Sulfide und/oder Dialkylamine können durch geeignete Oxidationsmittel (zum Beispiel Wasserstoffperoxid bzw. Persäuren) in die gewünschten Sulfoxide (n = 1), N-oxide

(n = 1) [siehe z.B. Kontakte (Darmstadt) 1986, 3, S. 12] bzw. Sulfone (n = 2) überführt werden.

Verbindungen mit einem Dialkylamin-Substituenten im 19,11β-Phenylenring können durch Reaktion mit Bromcyan in aprotischen Lösungsmitteln wie zum Beispiel Dioxan, Benzol oder Toluol bei erhöhter Temperatur (Amin-Abbau nach Braun) analog den zum Beispiel in Org. Reactions 7, 198 (1953), K.W. Bentley, Techniques of Organic Chemistry 11, 773 (1963) und Houben-Weyl, 5/4, 151 (1960) angegebenen Vorschriften in guter Ausbeute in die entsprechenden (N-Cyan-N-alkylaminoaryl)derivate überführt werden. Diese werden je nach der letztlich gewünschten Bedeutung von $R^{12}$ im Endprodukt in an sich bekannter Weise zu den entsprechenden Dialkylamin-Verbindungen (zum Beispiel mit Diisobutylaluminiumhydrid in Toluol zu den N-Formyl-N-alkylaminophenyl-Zwischenprodukten und anschließend mit Lithiumaluminiumhydrid) bzw. N-H-N-alkyl-Verbindungen reduziert (zum Beispiel mit Lithiumaluminiumhydrid oder mit Lithium in flüssigem Ammoniak). Die letzteren werden anschließend gewünschtenfalls in literaturbekannter Weise acyliert und gegebenenfalls anschließend in bekannter Weise mit zum Beispiel Lithiumaluminiumhydrid zum neuen Dialkylaminderivat reduziert (s. DE 36 23 038).

Die erhaltenen Verbindungen der allgemeinen Formel I mit X in der Bedeutung eines Sauerstoffatoms können gewünschtenfalls durch Umsetzung mit Hydroxylaminhydrochlorid in Gegenwart von tertiären Aminen bei Temperaturen zwischen -20 und +40 °C in die Oxime (Formel I mit X in der Bedeutung der Hydroxyiminogruppierung N OH, wobei die Hydroxygruppe syn- oder antiständig sein kann) überführt werden. Geeignete tertiäre Basen sind beispielsweise Trimethylamin, Triäthylamin, Pyridin, N,N-Dimethylaminopyridin, 1,5-Diazabicyclo[4.3.0]nonen5 (DBN) und 1,5-Diazabicyclo[5.4.0]undecen-5 (DBU), wobei Pyridin bevorzugt ist.

Die neuen Verbindungen der allgemeinen Formel I sowie deren Additionssalze mit pharmazeutisch verträglichen Säuren sind wertvolle Pharmaka. So verfügen sie über eine starke Affinität zum Gestagenrezeptor und besitzen eine überraschend großen Bereich an antigestagenen, antiglucocorticoiden und antimineralcorticoiden Eigenschaften. Diese wichtigen biologischen Wirksamkeiten können für medizinische Zwecke genutzt werden.

Wirkstoffe dieser Art mit ausgeprägter antigestagener Aktivität sin zur Auslösung von Aborten geeignet, da sie das zur Aufrechterhaltung der Schwangerschaft erforderliche Progesteron vom Rezeptor verdrängen. Sie sind deshalb wertvoll und interessant im Hinblick auf ihre Verwendung zur postcoitalen Fertilitätskontrolle.

Sie können auch gegen hormonelle Unregelmäßigkeiten, zur Menstruationsauslösung, zur Geburtseinleitung und zur Behandlung der Endometriose eingesetzt werden. Außerdem können sie für die Behandlung von hormonabhängigen Carcinomen eingesetzt werden.

Zur Bestimmung der antigestagenen Wirksamkeit der Verbindungen der allgemeinen Formel I wurde stellvertretend mit 9α,17β-Dihydroxy-17-(prop-1-inyl)-11β,19-[4-(3-pyridyl)-o-phenylen]-4-androsten-3-on der in der EP-A 0283 428, Seite 12, beschriebene Rattenabortivtest durchgeführt:

| Dosis s.c. mg/Tier/d | n Abort/ n behandelt |
|---|---|
| 3,0 | 4/4 |
| 1,0 | 4/4 |
| 0,3 | 2/4*) |

*) pathologische Nidationsstellen

| | |
|---|---|
| Kontrolle | |
| Benzylbenzoat/Rizinusöl (1:4) | 0/4 |

Die erfindungsgemäßen Verbindungen der allgemeinen Formel I sowie deren Additionssalze mit pharmazeutisch verträglichen Säuren weisen auch eine antiglucocorticoide Aktivität auf und können somit auch als Arzneimittel zur Therapie corticoid-induzierter Störungen (Glaukom) sowie zur Bekämpfung von Nebenwirkungen, die bei langfristiger Behandlung mit Glucocorticoiden auftreten (Cushing-Syndrom) eingesetzt werden. Sie ermöglichen daher auch die auf eine Supersekretion der Glucocorticoide zurückzuführenden Störungen, vor allem die Adipositas, Arteriosklerose, Hypertension, Osteoporose, Diabetes sowie die Insomnie zu bekämpfen.

Die erfindungsgemäßen Verbindungen der allgemeinen Formel I sowie deren Additionssalze pharmazeutisch verträglichen Säuren mit antimineralcorticoiden Eigenschaften können zur Behandlung von Krankheitszuständen, an denen ein Hyperaldosteronismus beteiligt ist, verwendet werden.

Die Erfindung betrifft somit auch Arzneimittel auf Basis der pharmazeutisch verträglichen, d.h. in den verwendeten Dosen nicht toxischen Verbindungen der allgemeinen Formel I sowie deren Additionssalze mit pharmazeutisch verträglichen Säuren, gegebenenfalls zusammen mit den üblichen Hilfs- und Trägerstoffen.

Die erfindungsgemäßen Verbindungen und deren Salze können nach an sich bekannten Methoden der Galenik zu pharmazeutischen Präparaten für die enterale, perkutane, parenterale oder lokale Applikation verarbeitet werden. Sie können in Form von Tabletten, Dragees, Gelkapseln, Granulaten, Suppositorien, Implantaten, injizierbaren sterilen wäßrigen oder öligen Lösungen, Suspensionen oder Emulsionen, Salben, Cremes und Gelen verabreicht werden.

Der oder die Wirkstoffe können dabei mit den in der Galenik üblichen Hilfsstoffen wie z.B. Gummiarabikum, Talk, Stärke, Mannit, Methylcellulose, Laktose, Tensiden wie Tweens$^{(R)}$ oder Myrj$^{(R)}$, Magnesiumstearat, wäßrigen oder nichtwäßrigen Trägern, Paraffinderivaten, Netz-, Dispergier-, Emulgier-, Konservierungsmitteln und Aromastoffen zur Geschmackskorrektur (z.B. ätherischen Ölen) gemischt werden.

Die Erfindung betrifft somit auch pharmazeutische Zusammensetzungen, die als Wirkstoff zumindest eine erfindungsgemäße Verbindung oder eines ihrer Additionssalze mit pharmazeutisch verträglichen Säuren enthalten.

Als Additionssalze der erfindungsgemäßen Produkte mit Säuren sind insbesondere die Hydrochloride und die Methansulfonate zu nennen.

Eine Dosiseinheit enthält etwa 1-100 mg Wirkstoff(e).

Die Dosierung der erfindungsgemäßen Verbindungen liegt beim Menschen bei etwa 1-1000 mg pro Tag.

Die folgenden Beispiele erläutern die Herstellung der erfindungsgemäßen Verbindungen.

## BEISPIEL 1

9α, 17β-Dihydroxy-11β,19-(4-ethyl-o-phenylen)-17-(prop-1-inyl)-4-androsten-3-on

a) 19-(2-Brom-5-methoxyphenyl)-3,3-(2,2-dimethyltrimethylendioxy)-9α,11α-epoxyandrostan-5α,17β-diol

Zu einer Lösung von 47,6 g (82,6 mmol) 19(2-Brom-5-methoxyphenyl)-3,3(2,2-dimethyltrimethylendioxy)-9,11-androsten-5α,17β-diol in 0,8 l Methylenchlorid werden bei Raumtemperatur nacheinander 225 ml einer 0.5 m wäßrigen Natriumhydrogencarbonatlösung und 22,6 g 67%ige m-Chlorperbenzoesäure zugegeben. Anschließend wird das Reaktionsgemisch 1,5 Stunden nachgerührt. Zur Aufarbeitung trennt man die wäßrige Phase ab, extrahiert sie mit wenig Methylenchlorid und vereinigt die organischen Phasen. Diese werden nacheinander mit gesättigter Natriumthiosulfatlösung, 5%iger Natronlauge und Wasser gewaschen, über Natriumsulfat getrocknet und am Vakuum eingeengt. Die Reinheit des Rohprodukts (47,4 g) ist ausreichend für die weitere Umsetzung unter b). Zur Charakterisierung werden 400 mg des Rohprodukts an Kieselgel mit einem Gemisch aus Ethylacetat/Hexan chromatographiert. Man erhält 357 mg 19(2-Brom-5-methoxyphenyl)-3,3-(2,2-dimethyltrimethylendioxy)-9α,11α-epoxyandrostan-5α,17β-diol.

b) 3,3(2,2-Dimethyltrimethylendioxy)-11β,19-(4-methoxy-o-phenylen)-androstan-5α,9α,17β-triol

41,4 g (70 mmol) 19-(2-Brom-5-methoxyphenyl)-3,3-(2,2-dimethyltrimethylendioxy)-9α,11α-epoxyandrostan-5α,17β-diol, gelöst in 350 ml absolutem Diethylether, werden unter Schutzgas bei Raumtemperatur zu 435 ml einer 0,8m etharischen Methyimagnesiumjodidlösung zugegeben. Nach 30-minütigem Nachrühren wird das Reaktionsgamisch mit 825 ml einer 1,6 m n-Butyllithiumlösung (Hexan) versetzt und über Nacht nachgerührt. Anschließend wird es vorsichtig auf eisgekühlte gasättigte wäßrige Ammoniumchloridlösung gegossen, die organische Phase abgetrennt und die wäßrige mit Ethylacatat nachaxtrahiert. Die organischen Phasen werden vereinigt, mit gesättigter, wäßriger Kochsalzlösung gewaschen, über Natriumsulfat getrocknet und am Vakuum eingeengt. Das Rohprodukt (37,2 g) wird an Kieselgel mit einem Gemisch aus Ethylacetat/Hexan chromatographiert und man erhält 31,3 g der Titelverbindung als weißen Schaum. Fp.: = 254-255° C (Ethylacetat)

c) 5α,9α-Dihydroxy-3,3-(2,2-dimethyltrimethylendioxy)-11β,19-(4-methoxy-o-phenylen)-androstan-17-on

Zu einem Gemisch aus 120 ml Pyridin und 875 ml Methylenchlorid werden bei 0 °C portionsweise 35,1 g Chromtrioxid zugegeben. Anschließend werden 30 g 3,3-(2,2-Dimethyltrimethylendioxy)-11β,19-(4-methoxy-o-phenylen)-androstan-5α,9α,17β-triol, gelöst in 100 ml Methylenchlorid, langsam bei der gleichen Temperatur zugetropft und das Reaktionsgemisch weitere 1,5 Stunden bei Eisbadtemperatur nachgerührt. Nach Beendigung des Rührens läßt man die festen Bestandteile sich absetzen, dekantiert die überstehende Phase ab und wäscht den Niederschlag mehrmals gründlich mit Methylenchlorid aus. Die vereinigten organischen Phasen werden durch Waschen mit 0,5 m Kaliumhydroxidlösung von restlichen anorganischen Bestandteilen befreit, mit Wasser neutral gewaschen, über Natriumsulfat getrocknet und am Vakuum eingeengt. Durch Chromatographie des Rückstandes an Keiselgel mit einem Gemisch aus Ethylacetat/Hexan werden 26,7 g der Titelverbindung als weißer Schaum isoliert.

d) 5α,9α-Dihydroxy-3,3-(2,2-dimethyltrimethylendioxy)-11β,19-(4-hydroxy-o-phenylen)-androstan-17-on

25,5 g (50 mml) 5α,9α-Dihydroxy-3,3-(2,2-dimethyltrimethylendioxy)-11β,19-(4-methoxy-o-phenylen)-androstan-17-on werden in 250 ml absolutem Dimethylformamid gelöst und unter Schutzgas mit 14 g Natriumthiomethylat versetzt. Unter Inertgasatmosphäre wird das Reaktionsgemisch 3 Stunden zum Rückfluß erhitzt, anschließend wird auf Raumtemperatur abgekühlt und dann auf 4 l Eiswasser gegossen. Es wird solange nachgerührt, bis das Rohprodukt als weißer Feststoff ausgeflockt ist. Anschließend wird es abgesaugt, mit viel Wasser gewaschen und am Vakuum getrocknet. Es werden 22,6 g der Titelverbindung als Rohprodukt erhalten, dessen Reinheit für die nachfolgenden Umsetzungen ausreichend ist.

e) 5α,9α-Dihydroxy-3,3-(2,2-dimethyltrimethylendioxy)-11β,19-(4-trifluormethansulfonyloxy-o-phenylen)-androstan-17-on

21,85 g (44 mmol) 5α,9α-Dihydroxy-3,3-(2,2-dimethyltrimethylendioxy)-11β,19-(4-hydroxy-o-phenylen)-androstan-17-on werden in 675 ml absolutem Methylenchlorid gelöst und mit 29,8 g 4-Dimethylaminopyridin versetzt. Unter Schutzgas wird die Lösung anschließend auf -70 °C gekühlt und durch langsames Zutropfen mit 9,7 ml Trifluormethansulfonsäureanhydrid gelöst in 60 ml absolutem Methylenchlorid versetzt. Nach 30-minütigem Nachrühren bei -70 °C wird das Reaktionsgemisch auf gesättigte wäßrige Natriumhydrogencarbonatlösung gegossen, die organische Phase abgetrennt und die wäßrige Phase mehrmals mit Methylenchlorid extrahiert. Die vereinigten organischen Phasen werden mit gesättigter Natriumchloridlösung gewaschen, über Natriumsulfat getrocknet und am Vakuum eingeengt. Man erhält nach Chromatographie des Rückstandes an Keiselgel mit einem Gemisch aus Ethylacetat/Hexan 23,1 g der Titelverbindung als weißen Schaum.

f) 5α,9α-Dihydroxy-3,3-(2,2-dimethyltrimethylendioxy)-11β,19-(4-vinyl-o-phenylen)-androstan-17-on

4 g 5α,9α-Dihydroxy-3,3-(2,2-dimethyltrimethylendioxy)-11β,19-(4-trifluormethansulfonyloxy-o-phenylen)-androstan-17-on werden in 64 ml absolutem Dimethylformamid gelöst und mit 542 mg Lithiumchlorid, 0,37 g Tetrakistriphenylpalladium und 2,34 ml Tributylvinylzinn versetzt. Anschließend wird das Reaktionsgemisch 1,5 Stunden bei 110 °C unter Schutzgas gerührt und dann auf Raumtemperatur abgekühlt. Nach Filtration über Celite und Waschen des Filterrückstandes mit Ethylacetat wird die organische Phase mit gesättigter Natriumchloridlösung gewaschen, über Natriumsulfat getrocknet und am Vakuum eingeengt. Chromatographie des Rückstandes an Keiselgel mit einem Gemisch aus Ethylacetat/Hexan ergibt 2,96 g der Titelverbindung als weißen Schaum.

g) 5α,9α-Dihydroxy-3,3-(2,2-dimethyltrimethylendioxy)-11β,19-(4-ethyl-o-phenylen)-androstan-17-on

1,5 g 5α,9α-Dihydroxy-3,3-(2,2-dimethyltrimethylendioxy)-11β,19-(4-vinyl-o-phenylen)-androstan-17-on werden in 15 ml absolutem Tetrahydrofuran gelöst und nach Zugabe von 1,5 ml Pyridin mit 150 mg Palladium auf Bariumsulfat (10%ig) als Katalysator bei Normaldruck hydriert. Nach Aufnahme eines Äquivalents Wasserstoff wird das Reaktionsgemisch über Celite abgesaugt, der Filterrückstand mit Essigester nachgewaaschen und das Filtrat am Vakuum eingeengt. Kristallisation des Rohproduktes aus Ethylacetat führt zu 1,26 g der Titelverbindung.

h) 3,3-(2,2-Dimethyltrimethylendioxy)-11β,19-(4-ethyl-o-phenylen)-17-(prop-1-inyl)-androstan-5α,9α,17β-triol

30 ml absolutes Tetrahydrofuran werden bei 0 °C mit Propin gesättigt. Anschließend werden zu dieser Lösung 3,7 ml einer 1,6 m-Butyllithiumlösung (Hexan) langsam ohne größere Temperaturerhöhung zugetropft. Nach 15-minütigem Nachrühren tropft man bei Eisbadkühlung langsam eine Lösung von 300 mg 5α,9α-Dihydroxy-3,3-(2,2-dimethyltrimethylendioxy)-11β,19-(4-ethyl-o-phenylen)-androstan-17-on, gelöst in 6 ml absolutem Tetrahydrofuran, zu dieser Reaktionsmischung und läßt sie über Nacht nachrühren. Danach wird das Reaktionsgemisch auf Wasser gegossen, die wäßrige Phase mit Ethylacetat extrahiert und die organische Phase mit Natriumchloridlösung gewaschen. Nach Trocknen über Natriumsulfat und Einengen der organischen Phase am Vakuum wird der Rückstand an Aluminiumoxid (neutral, Stufe III) chromatographiert. Es werden 295 mg der Titelverbindung als weißer Schaum erhalten.

i) 9α,17β-Dihydroxy-11β,19-(4-ethyl-o-phenylen)-17-(prop-1-inyl)-4-androsten-3-on

280 mg 3,3-(2,2-Dimethyltrimethylendioxy)-11β,19-(4-ethyl-o-phenylen)-17-(prop-1-inyl)-androstan-5α,9α,17β-triol werden in 20 ml Aceton gelöst und mit 0,1 ml 4 n wäßriger Salzsäure versetzt. Nach 3-stündigem Nachrühren bei 40 °C wird das Reaktionsgemisch auf gesättigte Natriumhydrogencarbonatlösung gegossen und die wäßrige Phase mit Methylenchlorid extrahiert. Die vereinigten organischen Phasen werden mit gesättigter Natriumchloridlösung gewaschen, über Natriumsulfat getrocknet und am Vakuum eingeengt. Man erhält nach Chromatographie des Rückstandes an Kieselgel mit einem Gemisch aus Ethylacetat/Hexan 234 mg der Titelverbindung als weißen Schaum. Fp.: = 155-160 °C (Hexan/Methylenchlorid)

**BEISPIEL 2**

9α,17β-Dihydroxy-11β,19-(4-vinyl-o-phenylen)-17-(prop-1-inyl)-4-androsten-3-on

a) 3,3-(2,2-Dimethyltrimethylendioxy)-11β,19-(4-vinyl-o-phenylen)-17-(prop-1--inyl)-androstan-5α,9α,17β-triol

Analog der unter Beispiel 1h) beschriebenen Vorschrift werden 1 g der unter 1f) hergestellten Ketoverbindung in die entsprechende 17-Propinylverbindung überführt. Es werden 0,97 g der obigen Verbindung als weißer Schaum erhalten.

b)9α,17β-Dihydroxy-11β,19-(4-vinyl-o-phenylen)-17-(prop-1-inyl)-4-androsten-3-on

Analog der unter Beispiel 1i) beschriebenen Vorschrift werden 0,9 g der unter 1d) hergestellten Propinylverbindung umgesetzt. Es werden 660 mg der Titelverbindung als weißer Schaum erhalten.
Fp.: = 171-175 °C (Diisopropylether/Methylenchlorid)

**BEISPIEL 3**

9α,17β-Dihydroxy-11β,19-[4-(3-pyridyl)-o-phenylen]-17-(prop-1-inyl)-4-androsten-3-on

a) 5α,9α-Dihydroxy-3,3-(2,2-dimethyltrimethylendioxy)-11β,19-(4-tri-n-butylstannyl-o-phenylen)-androstan-17-on

2,6 g des unter Beispiel 1e) hergestellten Triflats werden in 41 ml absolutem Dioxan gelöst und unter Schutzgas mit 6,2 ml Hexa-n-butylzinn, 521 mg Lithiumchlorid und 190 mg Tetrakistriphenylphosphinpalladium versetzt. Anschließend wird das Reaktionsgemisch auf 110 °C erwärmt, 1 Stunde nachgerührt, auf Raumtemperatur abgekühlt und am Vakuum eingeengt. Der Rückstand wird an Kieselgel mit einem Gemisch aus Ethylacetat/Hexan chromatographiert. Man erhält 2,75 g der obigen Verbindung als weißen Schaum.

b) 5α,9α-Dihydroxy-3,3-(2,2-dimethyltrimethylendioxy)-11β,19-[4-(3-pyridyl)-o-phenylen]-androstan-17-on

2,7 g des unter a) hergestellten Zinnorganyls werden in 41 ml absolutem Toluol gelöst, mit 190 mg Tetrakistriphenylphosphinpalladium und 4 ml 3-Brompyridin versetzt. Anschließend wird das Reaktionsgemisch 17 Stunden auf 110 °C erwärmt, auf Raumtemperatur abgekühlt und am Vakuum eingeengt. Der Rückstand wird an Kieselgel mit einem Gemisch aus Ethylacetat/Hexan chromatographiert. Man erhält 1,47 g der obigen Verbindung als weißen Schaum.

c) 3,3-(2,2-Dimethyltrimethylendioxy)-11β,19-[4-(3-pyridyl-o-phenylen]-17-(prop-1-inyl)-androstan-5α,9α,17β-triol

Analog der unter Beispiel 1h) beschriebenen Vorschrift werden 400 mg der unter b) hergestellten Ketoverbindung in die entsprechende 17-Propinylverbindung überführt. Es werden 402 mg der obigen Verbindung als weißer Schaum erhalten.

d) 9α,17β,Dihydroxy-11β,19-[4-(3-pyridyl)-o-phenylen]-17-(prop-1-inyl)-4-androsten-3-on

Analog der unter Beispiel 1i) beschriebenen Vorschrift werden 380 mg der unter c) hergestellten Propinylverbindung umgesetzt. Es werden 288 mg der Titelverbindung als weißer Schaum erhalten.
Fp.: = 203-205 °C (Diisopropylether)

**BEISPIEL 4**

9α,17β-Dihydroxy-11β,19-(4-methoxy-o-phenylen)-4-androsten-3-on

Analog der unter Beispiel 1i) beschreibenen Vorschrift werden 0,5 g der unter Beispiel 1b) hergestellten Hydroxyverbindung umgesetzt. Es werden 306 mg der Titelverbindung als weißer Schaum erhalten. Fp.: = 175-177 °C (Ethylacetat)

**BEISPIEL 5**

9α, 17β-Dihydroxy-11β, 19-(4-hydroxy-o-phenylen)-4-androsten-3-on

a) 3,3-(2,2-Dimethyltrimethylendioxy)-11β,19-(4-hydroxy-o-phenylen)-androstan-5α,9α,17β-triol

Analog der unter 1d) beschriebenen Vorschrift werden 2,5 g der unter Beispiel 1b) hergestellten Methoxyverbindung umgesetzt. Es werden nach Chromatographie an Kieselgel mit einem Gemisch aus Ethylacetat/Hexan 2,15 g Phenol als weißer Schaum erhalten.

9α,17β-Dihydroxy-11β,19-(4-hydroxy-o-phenylen)-4-androsten-3-on

Analog der unter Beispiel 1i) beschriebenen Vorschrift werden 750 mg des unter a) hergestellten

Phenols umgesetzt. Es werden 394 mg der Titelverbindung als weißer Schaum erhalten.
Fp.: = 146-148 °C (Ethylacetat)

**BEISPIEL 6**

9α,17β-Dihydroxy-11β,19-[4-(4-cyanophenyl)-o-phenylen]-4-androsten-3-on

a) 3,3-(2,2-Dimethyltrimethylendioxy)-11β,19-(4-trifluormethansulfonyloxy-o-phenylen)-androstan-5α,9α,17β-triol

Analog der unter Beispiel 1e) beschriebenen Vorschrift werden 1,25 g des unter Beispiel 5a) hergestellten Phenols umgesetzt. Es werden nach Chroma tographie an Kieselgel mit einem Gemisch aus Ethylacetat/Hexan 1,3 g Triflat als weißer Schaum erhalten.

b) 3,3-(2,2-Dimethyltrimethylendioxy)-11β,19-(4-tri-n-butylstannyl-o-phenylen)-androstan-5α,9α,17β-triol

Analog der unter 3a) beschriebenen Vorschrift werden 1,25 g des unter a) hergestellten Triflats umgesetzt. Es werden nach Chromatographie an Keiselgel mit einem Gemisch aus Ethylacetat/Hexan 1,53 g Zinnorganyl als weißer Schaum erhalten.

c) 3,3-(2,2-Dimethyltrimethylendioxy)-11β,19-[4-cyanophenyl)-o-phenylen]-androstan-5α,9α,17β-triol

Analog der unter Beispiel 3b) beschreibenen Vorschrift werden 1,5 g des unter b) hergestellten Zinnorganyls mit 4 g 4-Brombenzonitril umgesetzt. Es werden nach Chromatographie an Keiselgel mit einem Gemisch aus Ethylacetat/Hexan 0,73 g Kupplungsprodukt als weißer Schaum erhalten.

d) 9α,17β-Dihydroxy-11β,19-[4-(4-cyanophenyl)-o-phenylen]-4-androsten3-on

Analog der unter Beispiel 1i) beschreibenen Vorschrift werden 700 mg des unter c) hergestellten Benzonitrils umgesetzt. Es werden 512 mg der Titelverbindung als weißer Schaum erhalten.
Fp.: = 186-190 °C (Diisopropylether)

**BEISPIEL 7**

11β,19-(4-Dimethylamino-o-phenylen)-9α,17β-dihydroxy-17-(prop-1-inyl)-4-androsten-3-on

a) 19-(3-Dimethylaminophenyl)-3,3-(2,2-dimethyltrimethylendioxy)-9(11)-androsten-5 α,17β-diol

10 g 19-(2-Chlor-5-dimethylaminophenyl)-3,3-(2,2-dimethyltrimethylendioxy)-9(11)-androsten-5 α,17β-diol werden in 500 ml Methanol unter Schutzgas vor gelegt und nacheinander mit 5,8 g Ammoniumformiat und 2,5 g Palladium auf Kohle (10%) versetzt. Nach 18-stündigem Nachrühren bei Raumtemperatur wird das Reaktionsgemisch über Celite filtriert, das Filtrat am Vakuum eingeengt und der Rückstand in Methylenchlorid aufgenommen. Die organische Phase wird mit Wasser gewaschen, über Natriumsulfat getrocknet und am Vakuum eingeengt. Die Reinheit des Rohprodukts ist für weitere Reaktionen ausreichend. 100 mg der Substanz werden zur vollständigen Charakterisierung über Kielselgel mit einem Gemisch aus Hexan/Ethylacetat chromatographiert. Es werden 73 mg der obigen Verbindung als weißer Schaum isoliert.

b) 19-(2-Brom-5-dimethylaminophenyl)-3,3-(2,2-dimethyltrimethylendioxy)-9-(11)-androstan-5 α,17β-diol

9 g des unter a) hergestellten Rohprodukts werden bei 0 °C in 450 ml Methylenchlorid vogelegt und nacheinander mit 3,1 ml Triethylamin und 3,78 g N-Bromsuccinimid versetzt. Nach 45-minütigem Nachrühren bei Eisbadtemperatur wird gesättigte Natriumthiosulfatlösung zugesetzt und die wäßrige Phase mit Methylenchlorid extrahiert. Die vereinigten organischen Phasen werden mit Wasser gewaschen, über Natriumsulfat getrocknet und am Vakuum eingeengt. Der Rückstand wird über Kieselgel mit einem Gemisch aus Hexan/Ethylacetat chromatographiert. Man erhält 9,7 g der obigen Verbindung als weißen Schaum.

c) N-<4-Brom-3-[9α,11α-epoxy-5α,17β-dihydroxy-3,3-(2,2-dimethyltrimethylendioxy)-androstan-19-yl]-phenyl>-N,N-dimethylaminoxid

Zu einer Lösung von 8,2 g des unter b) dargestellten Bromids in 160 ml absolutem Methylenchlorid werden 100 ml einer 0,5 m Natriumhydrogencarbonatlösung und 18,2 g m-Chlorperbenzoesäure (67,7%ig) nacheinander zugegeben. Anschließend wird das Reaktionsgemisch bei Raumtemperatur 1 Stunde nachgerührt, dann mit 5%iger Natronlauge versetzt, die organische Phase abgetrennt und die wäßrige mit Methylenchlorid extrahiert. Die vereinigten organischen Phasen werden über Natriumsulfat getrocknet und am Vakuum eingeengt. Das erhaltene Rohprodukt (9,1 g) wird direkt in die nachfolgende Reaktion eingesetzt.

d) 11β,19-(4-Dimethylamino-o-phenylen)-3,3-(2,2-dimethyltrimethylendioxy)-androstan-5α,9α,17β-triol

Analog der unter Beispiel 1c) beschriebenen Vorschrift werden 9,1 g des unter c) erhaltenen Rohprodukts mit 140 ml einer 1 m etherischen Methylmagnesiumjodidlösung und 360 ml einer 1,6 m n-Butyllithiumlösung (Hexan) umgesetzt. Es werden nach Chromatographie an Kieselgel einem Gemisch aus Hexan/Ethylacetat 3,54 g der obigen Verbindung als weißer Schaum isoliert.
$^1$H-NMR ($CD_2Cl_2$) [δ] ppm: 7,18 (1H,d,J=9,5Hz); 6,59 (1H,dd,J=9,5 und $J_1$=2Hz); 6,39 (1H,d,$J_1$=2Hz); 5,87 (1H,s); 5,36 (1H,s); 3,68 (1H,m); 3,65-3,35 (4H,m); 3,15 (1H,d,$J_2$=17,5Hz); 2,94 (1H,m); 2,87 (6H,s); 2,75 (1H,d,$J_2$=17,5Hz); 1,02 (3H,s); 0,96 (3H,s); 0,22 (3H,s).

e) 11β,19-(4-Dimethylamino-o-phenylen)-3,3-(2,2-dimethyltrimethylendioxy)-5α,9α-dihydroxy-androstan-17-on

700 mg N-Chlorsuccinimid werden bei 0 °C in 70 ml absolutem Methylenchlorid vorgelegt. Nach Zutropfen von 0,49 ml Dimethylsulfid wird das Gemisch 30 minuten nachgerührt. Anschließend werden 3,5 g der unter d) hergestellten 17-Hydroxyverbindung gelöst in 35 ml absolutem Methylenchlorid langsam zugetropft. Nach 1,5-stündigem Nachrühren unter Feuchtigkeitsausschluß werden 0,91 ml Triethylamin zum Reaktionsgemisch zugageben. Dann wird es auf Wasser gegossen, die wäßrige Phase mit Methylenchlorid extrahiert und die organische Phase mit gesättigter Natriumchloridlösung gewaschen. Anschließend wird sie über Natriumsulfat getrocknet und zm Vakuum eingeengt. Nach Chromatographie des Rückstandes an Kieselgel mit einem Gemisch aus Hexan/Ethylacetat werden 2,98 g des obigen Ketons als weißer Schaum erhalten.

f) 11β,19-(4-Dimethylamino-o-phenylen)-3,3-(2,2-dimethyltrimethylendioxy)-17-(prop-1-inyl)-androstan-5α,9α-17β-triol

Analog der unter Beispiel 1h) beschriebenen Vorschrift werden 0,5 g der untr e) hergestellten Ketoverbindung in die entsprechende 17-Propinylverbindung überführt. Es werden 473 mg der obigen Verbindung als weißer Schaum isoliert.

g) 11β,19-(4-Dimethylamino-o-phenylen)-9α,17β-dihydroxy-17-(prop-1-inyl)-4-androstan-3-on

Analog der unter Beispiel 1i) beschriebenen Vorschrift werden 450 mg der unter g) hergestellten 17-Propinylverbindung in dei Titelverbindung überführt. Es werden 212 mg der obigen Verbindung als weißer Schaum isoliert.
$^1$H-NMR ($CDCl_3$) [δ] ppm: 7,23 (1H,d,J = 9,5Hz); 6,63 (1H,dd,J = 9,5 und $J_1$ = 2Hz); 6,42 (1H,d,$J_1$ = 2Hz); 5,99 (1H,s); 3,4 (1H,d,$J_2$ = 17,5Hz); 3,13 (1H,m); 2,93 (6H,s); 2,86 (1H,d,$J_2$ = 17,5Hz); 1,9 (3H,s); 0,4 (3H,s).

**BEISPIEL 8**

11β,19-(4-Ethyl-o-phenylen)-5α,9α-dihydroxyandrostan-3,17-dion

500 mg der unter Beispiel 1g) hergestellten Ketoverbindung werden in 10 ml 70%iger Essigsäure bei 0 °C zur Titelverbindung umgesetzt (ca. 60 Minuten Rühren). Das entstandene Reaktionsgemisch wird mit Wasser verdünnt und die wäßrige Phase mit Methlenchlorid extrahiert. Die vereinigten organischen Phasen werden nacheinander mit gesättigter Natriumhydrogencarbonatlösung und gesättigter Kochsalzlösung gewaschen und anschließend über Natriumsulfat getrocknet. Nach Einengen am Vakuum wird der Rückstand an Kieselgel chromatographiert. Es werden 212 mg der Titelverbindung als weißer Schaum isoliert.
Fp.: 155-160 °C (Ethylacetat)

9α,17β-Dihydroxy-17-methyl-11β,19-[4-(3-pyridyl)-o-phenyl]-4-androsten-3-on

a) 3,3-(2,2-Dimethyltrimethylendioxy)-17-metyhl-11β,19-[4-(3-pyridyl)-o-phenylen]-androstan-5α,9α,17β-triol

1,1 g 5α,9α-Dihydroxy-3,3-(2,2-dimethyltrimethylendioxy)-11β,19-[4-(3-pyridyl)-o-phenylen]-androstan-17-on gelöst in 15 ml absolutem Tetrahydrofuran werden langsam bei 0 °C zu 17,2 ml einer 1,6 m Lösung von Methyllithium in Diethylether zugetropft. Anschließend wird das Reaktionsgemisch auf eisgekühlte gesättigte Ammoniumchloridlösung gegossen und die wässrige Phase mit Essigester extrahiert. Nach Trocknen der vereinigten organischen Phasen über Natriumsulfat und Einengen am Vakuum wird der Rückstand an Keiselgel mit einem Gemisch aus Methanol/Ethylacetat chromatographiert. Man erhält 135 mg des Ausgangsmaterials zurück und 875 mg der obigen Verbindung als weißen Schaum.

b) 9α,17β-Dihydroxy-17-methyl-11β,19-[4-(3-pyridyl)-o-phenylen]-4-androsten-3-on

Analog der unter Beispiel 1 i) beschreibenen Vorschrift werden 800 mg der unter a) dargestellten Methylverbindung umgesetzt. Es werden 535 mg der Titelverbindung als weißer Schaum erhalten.
$^1$H-NMR ($CDCl_3$) [δ] ppm: 8,57 (1H, dd $J_1$ = 5Hz und $J_2$ = 0,5Hz); 7,88 (1H, d breit J = 8,5Hz); 7,28-7,55 (5H, m); 6,02 (1H, s); 3,54 (1H, d J = 17,5Hz); 3,23 (1H, d breit J = 5Hz); 2,99 (1H, d J = 17,5Hz); 1,3 (3H,s); 0,4 (3H, s)
$[\alpha]^{20}_D = +46°$ ($CHCl_3$;c = 0.505)

Beispiel 10

9α,17β-Dihydroxy-17-(3-hydroxyprop-1-inyl)-11β,19-[4-(3-pyridyl)-o-phenylen]-4-androsten-3-on

a) 3,3-(2,2-Dimethyltrimethylendioxy)-17-(3-hydroxyprop-1-inyl)-11β,19-[4-(3-pyridyl)-o-phenylen]-androstan-5α,9α,17β-triol

In 100 ml absolutem Tetrahydrofuran werden unter Schutzgas bei 0 °C 5,5 ml Propargylalkohol und 3,8 g 5α,9α-Dihydroxy-3,3-(2,2-dimethyltrimethylendioxy)-11β,19-[4-(3-pyridyl)-o-phenylen]-androstan-17-on vorgelegt, anschließend mit 15,7 g Kaliumethylat versetzt und das Reaktionsgemisch unter langsamer Erwärmung auf Raumtemperatur über Nacht nachgerührt. Anschließend wird es auf eisgekühlte gesättigte Ammoniumchloridlösung gegossen und die wässrige Phase mit Essigester extrahiert. Nach Trocknen der

vereinigten organischen Phasen über Natriumsulfat und Einengen am Vakuum wird der Rückstand an Kieselgel mit einem Gemisch aus Methanol/Ethylacetat chromatographiert. Man erhält 260 mg des Ausgangsmaterials zurück und 4,09 g der obigen Verbindung als weißen Schaum.

b) 9α,17β-Dihydroxy-17-(3-hydroxyprop-1-inyl)-11β,19-[4-(3-pyridyl)-o-phenylen]-4-androsten-3-on

Analog der unter Beispiel 1 i) beschriebenen Vorschrift werden 4 g der unter a) dargestellten Hydroxypropinylverbindung umgesetzt. Es werden 2,48 g der kristallinen Titelverbindung aus Ethylacetat erhalten.
Fp.: 206-208 °C (Ethylacetat)
$[\alpha]^{20}_D = +57°$ ($CHCl_3$;c=0.505)

Beispiel 11

9α,17β-Dihydroxy-17-(3-hydroxyprop-1-(Z)-inyl)-11β,19-[4-(3-pyridyl)-o-phenylen]-4-androsten-3-on

1,7 g 9α,17β-Dihydroxy-17-(3-hydroxyprop-1-inyl)-11β,19-[4-(3-pyridyl)-o-phenylen]-4-androsten-3-on werden in einem Gemisch aus 17 ml Tetrahydrofuran und 17 ml Ethanol gelöst, mit 1,7 ml Pyridin versetzt und unter Verwendung von 170 mg Palladium (10%-ig) auf Bariumsulfat als Katalysator bei Normaldruck hydriert. Nach Aufnahme eines Equivalents Wasserstoff wird das Reaktionsgemisch über Celite filtiert, der Filterückstand mit Ethylacetat nachgewaschen und das Filtrat am Vakuum eingeengt. Der Rückstand wird aus Ethanol umkristallisiert. Man erhält 1,3 g der Titelverbindung.
Fp.: 255-257 °C (Ethanol)
$[\alpha]^{20}_D = +92°$ ($CHCl_3$;c=0.505)

Beispiel 12

2′,5′-Dihydro-11β,19-[4-(3-pyridyl)-o-phenylen]-9α-hydroxy-spiro-[androst-4-en-17β,2′-furan]-3-on

Bei 0 °C werden 1,5 g 9α,17β-Dihydroxy-17-(3-hydroxyprop-1-(Z)-inyl)-11β,19-[4-(3-pyridyl)-o-phenylen]-4-androsten-3-on in 50 ml absolutem Dimethylformamid vogelegt und mit 4,3 ml Triethylamin versetzt. Nach Zugabe von 0,6 ml Methansulfonsäurechlorid wird das Reaktionsgemisch bei 0 °C 2 Stunden nachgerührt. Anschließend wird es auf gesättigte Natriumhydrogencarbonatlösung gegossen und die wässrige Phase mit Methylenchlorid extrahiert. Die vereinigten organischen Phasen werden mit gesättigter Natriumchloridlösung gewaschen, über Natriumsulfat getrocknet und am Vakuum eingeengt. Der Rückstand wird an Kieselgel mit einem Gemisch aus Ethylacetat/Hexan chromatographiert und man erhält 1,15 g der Titelverbindung.
$^1$H-NMR ($CDCl_3$) [δ] ppm: 8,85 (1H, s breit); 8,58 (1H, m); 7,88 (1H, m); 7,3-7,55 (5H, m); 6,03 (1H, s); 5,87-5,97 (2H, m); 4,55-4,7 (2H, m); 3,54 (1H, d J=17,5Hz); 0,45 (3H, s)

Beispiel 13

9α,17β-Dihydroxy-17-(ethinyl)-11β,19-[4-(3-pyridyl)-o-phenylen]-4-androsten-3-on

a) 3,3-(2,2-Dimethyltrimethylendioxy)-17-(ethinyl)-11β,19-[4-(3-pyridyl)-o-phenylen]-androstan-5α,9α,17β-triol

Analog der unter Beispiel 1 h) beschriebenen Vorschrift werden 2 g des unter Beispiel 3 c) dargestellten Ketons zur Ethinylverbindung umgesetzt. Es werden 1,98 g der obigen Verbindung als weißer Schaum erhalten.

<u>b) 9α,17β-Dihydroxy-17-(ethinyl)-11β,19-[4-(3-pyridyl)-o-phenylen]-4-androsten-3-on</u>

Analog der unter Beispiel 1 i) beschriebenen Vorschrift werden 1,9 g der unter a) dargestellten Ethinylverbindung umgesetzt. Es werden 1,28 g der kristallinen Titelverbindung aus Ethylacetat erhalten.
Fp.: 240-242 °C (Ethylacetat)
$[\alpha]^{20}_D = +92°$ ($CHCl_3$;c = 0.505)

## Ansprüche

1. 9α-Hydroxy-19,11β-überbrückte Steroide der allgemeinen Formel I

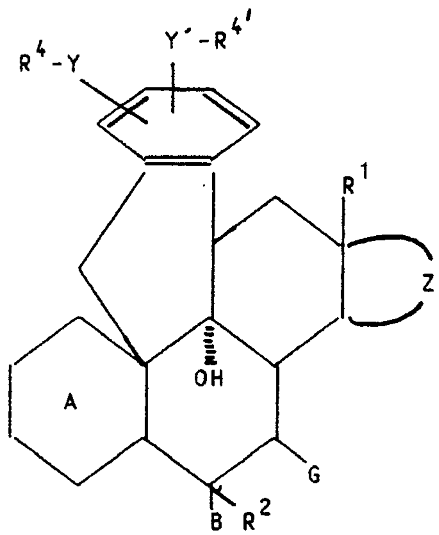

(I),

worin
$R^1$ für einen Methyl- oder Ethylrest,
$R^2$ für ein Wasserstoff-, Chloratom, einen $C_1$-$C_4$-Alkylrest,
B und G, die gleich oder verschieden sind, jeweils für ein Wasserstoffatom, einen $C_1$-$C_4$-Alkylrest oder gemeinsam für eine zweite Bindung zwischen den Kohlenstoffatomen 6 und 7,
B und $R^2$ gemeinsam für eine Methylen- oder Ethylengruppe,
Z für den Rest eines Ringes der Formel

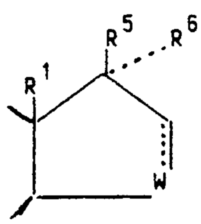

steht, worin
$R^1$ die in Formel I genannte Bedeutung hat,
die von W ausgehende gestrichelte Linie die etwaige Anwesenheit einer Doppelbindung bedeutet,
W einen $CH_2$-, CH-, $CH_2CH_2$- oder $CHCH_2$-Rest,
$R^5/R^6$
$-OR^{7'}/-C \equiv C-U$
$-OR^{7'}/-\underset{\underset{O}{\|}}{C}-CH_2-R^8$
$-\underset{\underset{O}{\|}}{C}-CH_2-R^8\ -OR^7$

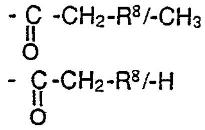

$-OR^7/-(CH_2)_m-CH_2-R^9$
$-OR^7/-CH=CH(CH_2)_k-CH_2-R^9$
$-OR^{10}/-H$
$-OR^{10}/-(CH_2)_k-C\equiv C-U$

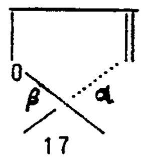 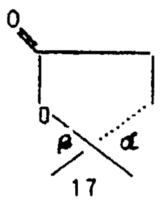 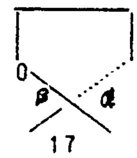

mit $R^7$ in der Bedeutung eines Wasserstoffatoms oder Acylrestes mit 1 bis 4 Kohlenstoffatomen, U in der Bedeutung eines Wasserstoffatoms, einer Alkyl-, Hydroxyalkyl-, Alkoxyalkyl-, Acyloxyalkylgruppe mit jeweils 1 bis 4 Kohlenstoffatomen im Alkyl- bzw. Acylrest oder eines Halogenatoms,
$R^8$ in der Bedeutung eines Wasserstoffatoms, einer Hydroxygruppe, einer Alkyl-, O-Alkyl- oder O-Acylgruppe mit jeweils 1 bis 4 Kohlenstoffatomen,
$R^9$ in der Bedeutung eines Wasserstoffatoms, eines Hydroxy- oder Cyanidrestes, einer O-Alkyl- oder O-Acylgruppe mit jeweils 1 bis 4 Kohlenstoffatomen,
$R^{10}$ in der Bedeutung eines Wasserstoffatoms, einer Alkyl- oder Acylgruppe mit jeweils 1 bis 10 Kohlenstoffatomen,
m in der Bedeutung 0, 1, 2 oder 3,
k in der Bedeutung 0, 1 oder 2,
bedeuten,
worin
$R^4$ und $R^{4'}$, die gleich oder verschieden sind, jeweils für ein Wasserstoffatom, einen Cyanidrest, eine $-OR^{11}$-, $-S(O)_kR^{11}$-, $-N(O)_nR^{11}R^{12}$-, $-O-SO_2R^{13}$-, $-P(O)(OR^{14})_2$, $-SiR^{14}_3$ oder $-SnR^{14}_3$ -Gruppe steht mit k in der Bedeutung der Ziffern 0, 1 oder 2, n in der Bedeutung der Ziffern 0 oder 1,
$R^{11}$ in der Bedeutung eines Wasserstoffatoms oder eines $C_1$-$C_8$-Alkylrestes,
$R^{12}$ in der Bedeutung von $R^{11}$, eines Cyanid- oder eines $C_1$-$C_{10}$-Acylrestes,
$R^{13}$ in der Bedeutung eines perfluorierten $C_1$-$C_4$-Alkylrestes,
$R^{14}$ in der Bedeutung eines $C_1$-$C_4$-Alkylrestes oder
$R^{11}$ und $R^{12}$ innerhalb einer $-N(O)_nR^{11}R^{12}$-Gruppe gemeinsam unter Einschluß von N einen 5- oder 6-gliederigen heterocyclischen Ring bilden, wobei im Ring noch ein weiteres Heteroatom N, O oder S enthalten sein kann,
Y und Y', die gleich oder verschieden sind, jeweils eine direkte Bindung, eine geradkettige oder verzweigte, gegebenenfalls Doppel- oder Dreifachbindung(en) aufweisende Alkylengruppe mit bis zu 20 Kohlenstoffatomen, die gegebenenfalls substituiert ist durch eine oder mehrere Oxo-, $C_1$-$C_{10}$-Acyloxy-, $-OR^{11}$-, $-S(O)_kR^{11}$- und/oder $-N(O)_nR^{11}R^{12}$-Gruppe(n), einen gegebenenfalls substituiertem Arylenrest oder
$R^4$-Y und $R^{4'}$-Y' gemeinsam den Rest eines, gegebenenfalls substituierten gesättigten, ungesättigten oder aromatischen 5-oder 6-gliedrigen Ringes mit 0 bis 2 Sauerstoff-, Schwefelatomen und/oder $NR^{11}$-Gruppen bedeuten, mit der Maßgabe, daß nur dann k und n größer als 0 sind, wenn $R^{11}$ für einen $C_1$-$C_8$-Alkylrest steht und
der Ring A für

a)

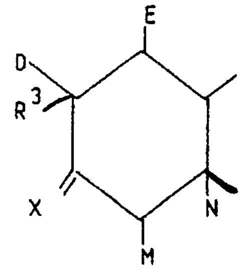

,

wobei M und N gemeinsam eine zweite Bindung
oder M ein Wasserstoffatom und N eine Hydroxygruppe, wobei dann B, $R^2$, G, $R^3$ D und E Wasserstoffatome sind
und X eine Sauerstoffatom, zwei Wasserstoffatome oder eine Hydroxyiminogruppierung N OH,
$R^3$ und D, die gleich oder verschieden sind, jeweils ein Wasserstoffatom, einen Nitrilrest oder einen $C_1$-$C_4$-Alkylrest oder gemeinsam eine Methylen-oder Ethylengruppe,
E ein Wasserstoffatom, einen $C_1$-$C_4$-Alkylrest,
D und E gemeinsam eine zweite Bindung zwischen den Kohlenstoffatomen 1 und 2 oder gemeinsam eine Methylengruppe
bedeuten
oder

b)

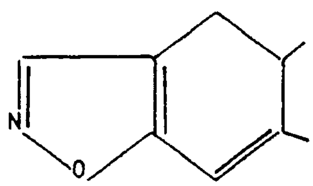

oder

c)

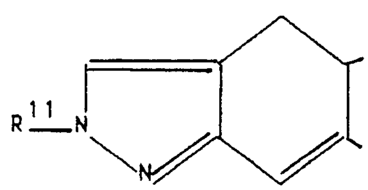

mit $R^{11}$ in der Bedeutung eines Wasserstoffatoms oder eines $C_1$-$C_8$-Alkylrestes stehen, sowie gegebenenfalls deren pharmazeutisch verträgliche Additionssalze mit Säuren.

2. Verbindungen nach Anspruch 1, dadurch gekennzeichnet, daß $R^2$, B und G jeweils für ein Wasserstoffatom stehen.

3. Verbindungen nach Anspruch 1, dadurch gekennzeichnet, daß B und G gemeinsam für eine zweite Bindung und $R^2$ für ein Wasserstoffatom stehen.

4. Verbindungen nach Anspruch 1, dadurch gekennzeichnet, daß Y und Y′ jeweils für eine direkte Bindung und $R^4$ und $R^4$ jeweils für eine Wasserstoffatom stehen.

5. Verbindungen nach Anspruch 1, dadurch gekennzeichnet, daß Y und Y′ jeweils für eine direkte Bindung, $R^4$ für ein Wasserstoffatom und $R^4$ für ein Stickstoffatom, substituiert mit zwei $C_1$-$C_8$-Alkylresten, stehen.

6. Verbindungen nach Anspruch 1, dadurch gekennzeichnet, daß Y und Y′ jeweils für eine direkte Bindung, $R^4$ für ein Wasserstoffatom und $R^4$ für eine $C_1$-$C_8$-Alkoxygruppe stehen.

7. Verbindungen nach Anspruch 1, dadurch gekennzeichnet, daß Y für eine direkte Bindung, $R^4$ und $R^4$

jeweils für ein Wasserstoffatom und Y′ für eine geradkettige oder verzweigte, gegebenenfalls Doppel- und/oder Dreifachbindung(en) aufweisende Alkylengruppe mit bis zu 20 Kohlenstoffatomen, die durch eine Oxo- oder $OR^{11}$-Gruppe mit $R^{11}$ in der Bedeutung eines Wasserstoffatoms oder eines $C_1$-$C_8$-Alkylrestes, substituiert ist, stehen.

8. Verbindungen nach Anspruch 1, dadurch gekennzeichnet, daß $R^4$-Y und $R^{4'}$-Y′ gemeinsam für den Rest eines gesättigten, ungesättigten oder aromatischen 5- oder 6-gliedrigen Ringes mit 0 bis 2 Sauerstoff-, Schwefelatomen und/oder $NR^{11}$-Gruppen mit $R^{11}$ in der Bedeutung eines Wasserstoffatoms oder eines $C_1$-$C_8$ Alkylrestes stehen.

9. Verbindungen nach Anspruch 1, dadurch gekennzeichnet, daß Y′-$R^{4'}$ ein Wasserstoffatom und Y-$R^4$ eine Ethyl-, Vinyl-, Ispropyl-,Isopropenyl-, Prop-1(Z)-einyl-, Prop-1(E)-enyl-, Prop-2-enyl-, Ethinyl-, Propinyl-, Prop-2-inyl-Methoxy-, Thiomethyl-, Thioethyl-, 1-Hydroxyethyl- oder Diethoxyphosphorylgruppe, ein substituierter oder unsubstituierter carbocyclischer oder heterocyclischer Arylrest ist.

10. Verbindungen nach Anspruch 9, dadurch gekennzeichnet, daß der Arylrest ein Phenyl-, Naphthyl-, 2-Methoxyphenyl-, 3-Methoxyphenyl-, 4-Methoxyphenyl-, 2-Tolyl-, 3-Tolyl-, 4-Tolyl-, 2-Dimethylamino-, 3-Dimethylamino-, 4-Dimethylamino-, Furyl-2-, Furyl-3-, Thienyl-2-, Thienyl-3-, Pyridyl-2-, Pyridyl-3-, Pyridyl-4-, Pyrimidinyl-, Thiazolyl-, Imidazolyl-Rest ist.

11.Verbindungen der allgemeinen Formel II

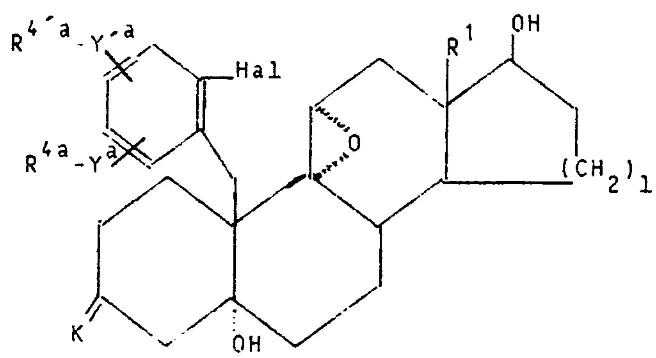

(II),

worin
$R^1$ einen methyl- oder Ethylrest
l die Ziffern 1 oder 2,
K eine in Form des Ketals oder Thioketals blockierte Ketogruppe bedeuten, Hal ein Chlor-, Brom- oder Jodatom bedeutet,
$R^{4a}$, $R^{4'a}$, $Y^a$ und $Y'^a$ unter Ausschluß des Cyanidrestes die gleiche Bedeutung wie $R^4$, $R^{4'}$, Y und Y′ haben, wobei gegebenenfalls vorhandene Hydroxy-, Mercapto-, Amino-, Oxo- und/oder terminale Acetylengruppen geschützt sind.

12. 9α-Hydroxy-19,11β-überbrückte Steroide der allgemeinen Formel IV

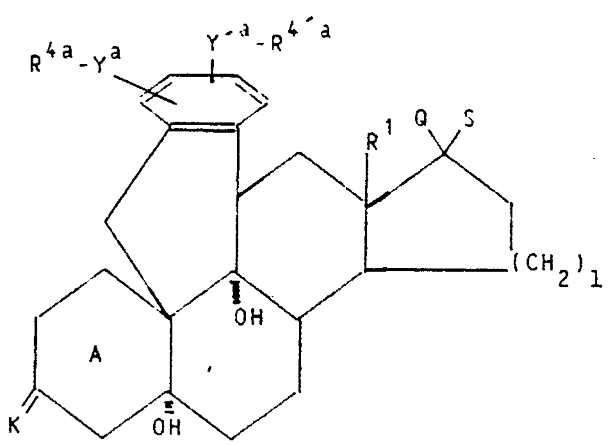

(IV),

worin
$R^1$ einen Methyl- oder Ethylrest,
I die Ziffern 1 oder 2,
K eine in Form des Ketals oder Thioketals blockierte Ketogruppe bedeuten und
$R^{4a}$, $R^{4\,a}$, $Y^{a}$ unter Ausschluß des Cyanidrestes die gleiche Bedeutung wie $R^4$, $R^4$, Y und Y′ haben, wobei gegenbenenfalls vorhandene Hydroxy-, Mercapto-, Amino-, Oxo- und/oder terminale Acetylengruppen geschützt sind, sowie Q für $R^5$ und S für $R^6$ oder aber gemeinsam für ein Ketosauerstoffatom stehen.

13.Verfahren zur Herstellung von Verbindungen der allgemeinen Formel I

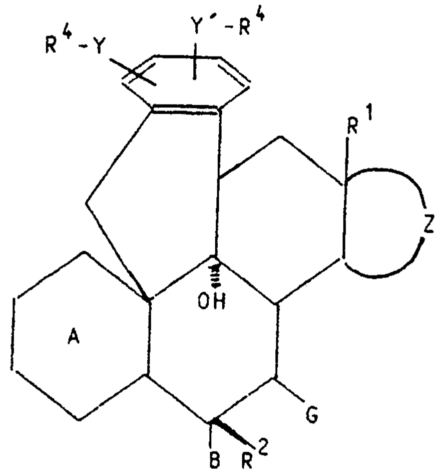

(I),

worin
$R^1$ für einen Methyl- oder Ethylrest,
$R^2$ für ein Wasserstoff-, Chloratom, einen $C_1$-$C_4$-Alkylrest,
B und G, die gleich oder verschieden sind, jeweils für ein Wasserstoffatom, einen $C_1$-$C_4$-Alkylrest oder gemeinsam für eine zweite Bindung zwischen den Kohlenstoffatomen 6 und 7,
B und $R^2$ gemeinsam für eine Methylen- oder Ethylengruppe,
Z für den Rest eines Ringes der Formel

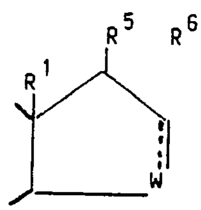

steht, worin
$R^1$ die in Formel I genannte Bedeutung hat,
die von W ausgehende gestrichelte Linie die etwaige Anwesenheit einer Doppelbindung bedeutet,
W einen $CH_2$-, CH-, $CH_2CH_2$- oder $CHCH_2$-Rest,
$R^5$/$R^6$
$-OR^7$/$-C \equiv C-U$
$-OR^7$/$-\underset{\underset{O}{\|}}{C}-CH_2-R^8$
$-\underset{\underset{O}{\|}}{C}-CH_2-R^8$/$-OR^7$
$-\underset{\underset{O}{\|}}{C}-CH_2-R^8$/$-CH_3$
$-\underset{\underset{O}{\|}}{C}-CH_2-R^8$/$-H$
$-OR^7$/$-(CH_2)_m-CH_2-R^9$

$-OR^7/-CH=CH(CH_2)_k-CH_2-R^9$
$-OR^{10}/-H$
$-OR^{10}/-(CH_2)_k-C \equiv C-U$

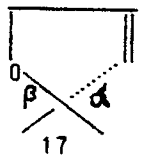
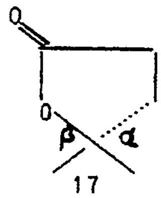
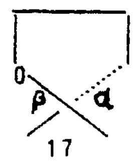

mit $R^7$ in der Bedeutung eines Wasserstoffatoms oder Acylrestes mit 1 bis 4 Kohlenstoffatomen, U in der Bedeutung eines Wasserstoffatoms, einer Alkyl-, Hydroxyalkyl-, Alkoxyalkyl-, Acyloxyalkylgruppe mit jeweils 1 bis 4 Kohlenstoffatomen im Alkyl- bzw. Acylrest oder eines Halogenatoms,

$R^8$ in der Bedeutung eines Wasserstoffatoms, einer Hydroxygruppe, einer Alkyl-, O-Alkyl- oder O-Acylgruppe mit jeweils 1 bis 4 Kohlenstoffatomen,

$R^9$ in der Bedeutung eines Wasserstoffatoms, eines Hydroxy- oder Cyanidrestes, einer O-Alkyl- oder O-Acylgruppe mit jeweils 1 bis 4 Kohlenstoffatomen,

$R^{10}$ in der Bedeutung eines Wasserstoffatoms, einer Alkyl- oder Acylgruppe mit jeweils 1 bis 10 Kohlenstoffatomen,

m in der Bedeutung 0, 1, 2 oder 3,

k in der Bedeutung 0, 1 oder 2,

bedeuten, ,

$R^4$ und $R^{4'}$, die gleich oder verschieden sind, jeweils für ein Wasserstoffatom, einen Cyanidrest, eine $-OR^{11}$-, $-S(O)_kR^{11}$-, $-N(O)_nR^{11}R^{12}$-, $-O-SO_2R^{13}$-, $-P(O)(OR^{14})_2$, $-SiR^{14}_3$ oder $-SnR^{14}_3$ -Gruppe steht mit k in der Bedeutung der Ziffern 0, 1 oder 2, n in der Bedeutung der Ziffern 0 oder 1,

$R^{11}$ in der Bedeutung eines Wasserstoffatoms oder eines $C_1$-$C_8$-Alkylrestes,

$R^{12}$ in der Bedeutung von $R^{11}$, eines Cyanid- oder eines $C_1$-$C_{10}$-Acylrestes,

$R^{13}$ in der Bedeutung eines perfluorierten $C_1$-$C_4$-Alkylrestes,

$R^{14}$ in der Bedeutung eines $C_1$-$C_4$-Alkylrestes oder

$R^{11}$ und $R^{12}$ innerhalb einer $-N(O)_nR^{11}R^{12}$-Gruppe gemeinsam unter Einschluß von N einen 5- oder 6-gliedrigen heterocyclischen Ring bilden, wobei im Ring noch eine weiteres Heteroatom N, O oder S enthalten sein kann,

Y und Y', die gleich oder verschieden sind, jeweils eine direkte Bindung, eine geradkettige oder verzweigte, gegebenenfalls Doppel- oder Dreifachbindung(en) aufweisende Alkylengruppe mit bis zu 20 Kohlenstoffatomen, die gegebenenfalls substituiert ist durch eine oder mehrere Oxo-, $C_1$-$C_{10}$-Acyloxy-, $-OR^{11}$-, $-S(O)_kR^{11}$- und/oder $-N(O)_nR^{11}R^{12}$-Gruppe(n), einen gegebenenfalls substituierten Arylenrest oder

$R^4$-Y und $R^{4'}$-Y' gemeinsam den Rest eines, gegebenenfalls substituierten gesättigten, ungesättigten oder aromatischen 5-oder 6-gliedrigen Ringes mit 0 bis 2 Sauerstoff-, Schwefelatomen und.oder $NR^{11}$-Gruppen bedeuten, mit der Maßgabe, daß nur dann k und n größer als 0 sind, wenn $R^{11}$ für einen $C_1$-$C_8$-Alkylrest steht und

der Ring A für

a)

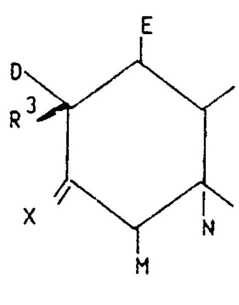

,

wobei M und N gemeinsam eine zweite Bindung

oder M ein Wasserstoffatom und N eine Hydroxygruppe, wobei dann B, $R^2$, G, $R^3$ D und E Wasserstoffato-

me sind
und X ein Sauerstoffatom, zwei Wasserstoffatome oder eine Hydroxyiminogruppierung N~OH,
$R^3$ und D, die gleich oder verschieden sind, jeweils ein Wasserstoffatom, einen Nitrilrest oder einen $C_1$-$C_4$-Alkylrest oder gemeinsam eine Methylen-oder Ethylengruppe,
E ein Wasserstoffatom, einen $C_1$-$C_4$-Alkylrest,
D und E gemeinsam eine zweite Bindung zwischen den Kohlenstoffatomen 1 und 2 oder gemeinsam eine Methylengruppe
bedeuten
oder

b)

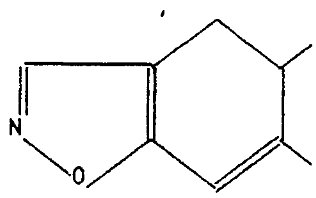

oder

c)

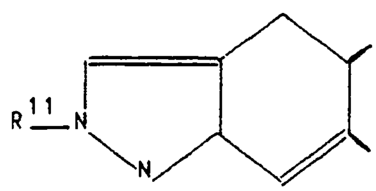

mit $R^{11}$ in der Bedeutung eines Wasserstoffatoms oder eines $C_1$-$C_8$-Alkylrestes stehen, sowie gegebenenfalls deren pharmazeutisch verträgliche Additionssalze mit Säuren,
dadurch gekennzeichnet, daß man eine Verbindung der allgemeinen Formel III

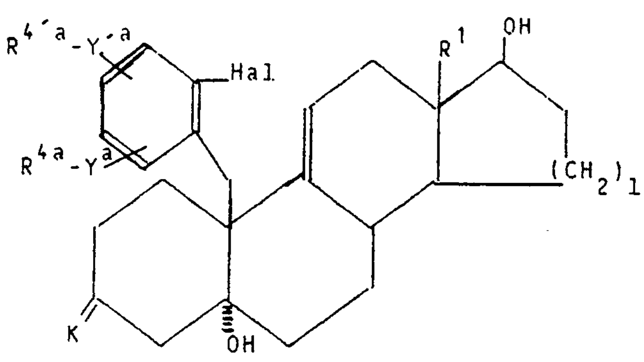

(III),

worin
$R^1$ einen Methyl- oder Ethylrest,
l die Ziffern 1 oder 2,
K eine in Form des Ketals oder Thioketals blockierte Ketogruppe bedeuten und
Hal ein Chlor-, Brom- oder Jodatom bedeutet,
$R^{4a}$, $R^{4'a}$, $Y^a$ und $Y'^a$ unter Ausschluß des Cyanidrestes die gleiche Bedeutung wie $R^4$, $R^{4'}$, Y und Y' haben, wobei gegebenenfalls vorhandene Hydroxy-, Mercapto-, Amino-, Oxo- und/oder terminale Acetylengruppen geschützt sind, zu einer Verbindung der allgemeinen Formel II

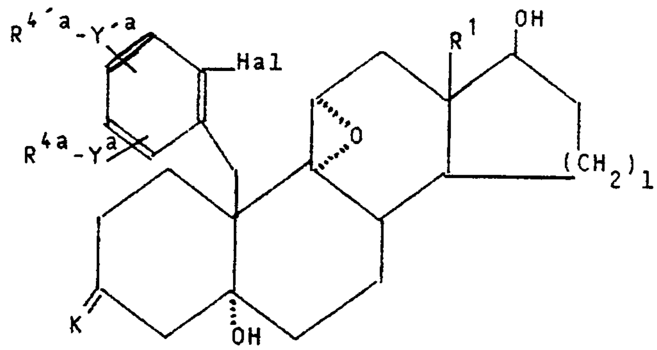

(II),

worin $R^1$, l, K, Hal sowie $R^{4a}$, $Y^a$, $R^{4'a}$, $Y'^a$ dieselbe Bedeutung wie in Formel I haben, epoxidiert, die epoxidierte Verbindung II dann zu einem Zwischenprodukt der allgemeinen Formel IVa cyclisiert

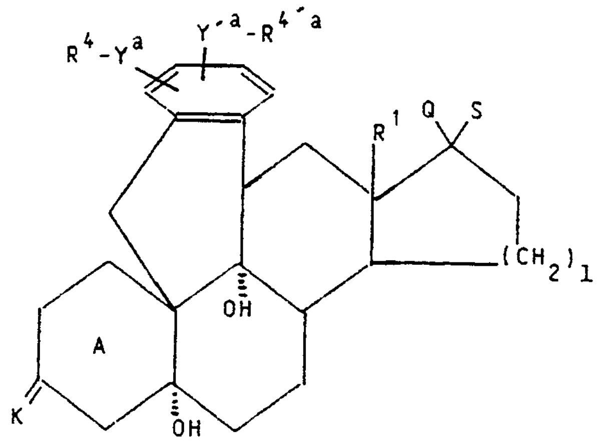

(IVa),

worin,

$R^{4a}$, $^{4'a}$, $Y^a$ und $Y'^a$ unter Ausschluß des Cyanidrestes die gleiche Bedeutung wie $R^4$, $R^{4'}$, Y und Y' haben, wobei gegebenenfalls vorhandene Hydroxy-, Mercapto-, Amino-, Oxo- und/oder terminale Acetylengruppen geschützt sind, sowie Q ausschließlich eine β-Hydroxygruppe und S ausschließlich ein α-Wasserstoffatom bedeuten, und dann entweder zuerst

a) die C-17-Hydroxygruppe gegebenenfalls oxidiert und anschließend

b) gegebenenfalls eine eine Schutzgruppe aufweisende Hydroxygruppe im 19,11β-Phenylenring von dieser Schutzgruppe befreit, gewünschtenfalls aus der Hydroxyverbindung ein entsprechendes Perfluoralkylsulfonat herstellt, gegebenenfalls das Perfluoralkylsulfonat entweder direkt oder durch Austausch der Perfluoralkylsulfonatabgangsgruppe gegen eine Zinntrialkylgruppe über die entsprechende Zinntrialkylverbindung in eine Verbindung überführt, die im 19,11β-Phenylenring, gegenbenenfalls nach weiteren Reaktionen, das gewünschte Substitutionsmuster aufweist, oder zuerst b) und dann a) ausführt und danach

c) den Ring D in gewünschter Weise nach an sich bekannten Methoden funktionalisiert, das so erhaltene Produkt der Einwirkung eines Dehydratationsmittels, das auch zur Freisetzung der 3-Oxogruppe befähig ist, zur Wasserabspaltung unter gleichzeitiger Ausbildung der 4(5)-Doppelbindung unterwirft, wobei gewünschtenfalls die Freisetzung der 3-Oxogruppe und die Wasserabspaltung nacheinander stattfinden und anschließend, gegebenenfalls nach erneutem Schutz von intermediär freigesetzten, im 19,11β-Phenylenring und/oder Z enthaltenen funktionellen Gruppen, die gewünschten Funktionen der Ringe A und B des Steroidgerüstes einführt oder

d) das so erhaltene Produkt der Einwirkung eines Dehydratationsmittels, das auch zur Freisetzung der 3-Oxogruppe befähigt ist zur Wasserabspaltung unter gleichzeitiger Ausbildung der 4(5)-Doppelbindung unterwirft, die gewünschten Funktionen der Ringe A und B des Steroidgerüstes einführt und anschließend, nach Schutz der 3-Oxogruppe, den Ring D in gewünschter Weise funktionalisiert,

oder die Schritte a) und b) nach dem Schritt c) oder d) ausführt, das so erhaltene Produkt gegebenenfalls von Schutzgruppen befreit, gewünschtenfalls die im 19,11β-Phenylenring gegebenenfalls enthaltene(n)

Hydroxy-, Mercapto -und/oder Aminogruppe(n) alkyliert bzw. acyliert, gewünschtenfalls einen Cyanidrest in den/die Arylsubstituenten einführt, gewünschtenfalls die in dem(n) Arylsubstituenten gegebenenfalls enthaltene(n) Amino- und/oder Sulfidgruppe(n) oxidiert, gewünschtenfalls mit Hydroxylamin-hydrochlorid zum Produkt der allgemeinen Formel I mit X in der Bedeutung der Hydroxyiminogruppierung N~OH umsetzt sowie gegebenenfalls ein pharmazeutisch verträgliches Säureadditinssalz herstellt.

14. Pharmazeutische Präparate, gekennzeichnet durch einen Gehalt an Verbindungen gemäß den Ansprüchen 1 bis 12.

15. Verwendung von Verbindungen gemäß den Ansprüchen 1 bis 12 zur Herstellung von Arzneimitteln.

Europäisches Patentamt

# EUROPÄISCHER RECHERCHENBERICHT

Nummer der Anmeldung

EP 90 25 0134

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 283 428 (SCHERING AG)<br>* Ganzes Dokument *<br>--- | 1 | C 07 J 53/00<br>C 07 J 71/00<br>A 61 K 31/56 |
| A | DE-A-3 717 169 (SCHERING AG)<br>* Ganzes Dokument *<br>----- | 1 | |

| RECHERCHIERTE SACHGEBIETE (Int. Cl.5) |
|---|
| C 07 J 53/00<br>C 07 J 71/00<br>A 61 K 31/00 |

Der vorliegende Recherchenbericht wurde für alle Patentansprüche erstellt

| Recherchenort | Abschlußdatum der Recherche | Prüfer |
|---|---|---|
| DEN HAAG | 06-08-1990 | WATCHORN P.W. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)